# EUROPEAN PATENT SPECIFICATION

(11) **EP 4 194 747 B1**
(45) Date of publication and mention of the grant of the patent: **30.04.2025**
(21) Application number: 20947878.3
(22) Date of filing: 04.08.2020
(51) Int. Cl.: H05B 47/17, H05B 47/155, H05B 47/16, H05B 47/165, H05B 45/20, A61N 5/06, F21W 131/20, F21V 14/00, F21W 131/205, F21W 131/208, F21V 5/00, F21Y 107/50, F21Y 113/10

(54) **MEDICAL SUSPENSION BRIDGE, LIGHTING SYSTEM, AND LIGHTING CONTROL METHOD**
MEDIZINISCHE HÄNGEBRÜCKE, BELEUCHTUNGSSYSTEM UND BELEUCHTUNGSSTEUERUNGSVERFAHREN
PONT DE SUSPENSION MÉDICALE, SYSTÈME D'ÉCLAIRAGE ET PROCÉDÉ DE COMMANDE D'ÉCLAIRAGE

(43) Date of publication of application: 14.06.2023
(73) Proprietor: Nanjing Mindray Bio-Medical Electronics Co., Ltd., Nanjing, Jiangsu 211111 (CN); Bartenbach Holding GmbH, 6071 Aldrans (AT)
(72) Inventor: WANG, Lei, Nanjing, Jiangsu 211111 (CN); YANG, Zhengyun, Nanjing, Jiangsu 211111 (CN); HU, Zhifeng, Nanjing, Jiangsu 211111 (CN); WEI, Zhengtang, Nanjing, Jiangsu 211111 (CN); JI, Changsheng, Nanjing, Jiangsu 211111 (CN); JIANG, Yajuan, Nanjing, Jiangsu 211111 (CN); DAI, Xiaoyu, Nanjing, Jiangsu 211111 (CN); CAO, Xudong, Nanjing, Jiangsu 211111 (CN)
(74) Representative: KIPA AB
(86) International application number: PCT/CN2020/106914
(87) International publication number: WO 2022/027256

(56) References cited:
- CN-A- 101 418 922
- CN-A- 101 910 721
- CN-A- 102 474 955
- CN-U- 207 999 708
- KR-U- 20120 006 182
- US-A1- 2005 265 024
- US-A1- 2018 318 601

## Description

### TECHNICAL FIELD

The disclosure relates to the technical field of medical devices, and more particularly to a medical suspension device, a lighting system, and a lighting control method.

### BACKGROUND

Take the medical suspension device as an example. The medical suspension device is used in an operating room, ICU (Intensive Care Unit), medical room, rehabilitation room, general ward and other medical places to provide a good operation environment and nursing space. Currently, the lighting for ICU is mainly provided by indoor lighting fixtures. When patients need to be examined, medical staff adds additional examination lamp. In addition, when multiple patient supports are placed in one medical room, the light of the examination lamp illuminates areas of other patient supports when the patient on one patient support is examined, this affects the patients in other areas.

US20180318601A1 discloses a circadian effect light fixture. The circadian effect light fixture includes a controller to receive a modifying factor based on a circadian impact profile. The modifying factor includes an indication of an intensity value, a correlated colour temperature value, and a colour value over a range of time. The circadian effect light fixture also includes a first lighting element controlled by the controller to produce a first light output having a first intensity, a first correlated colour temperature, and a first colour value. Further, the circadian effect light fixture includes a second lighting element controlled by the controller to produce a second light output having a second intensity, a second correlated colour temperature, and a second colour value. A combined light output of the first lighting element and the second lighting element tracks the modifying factor over the range of time.

### SUMMARY

In view of this, the embodiments of this disclosure expect to provide a medical suspension device, a lighting system and a lighting control method, wherein the lighting system is configured to provide lighting for an area of a single patient support.

A first aspect of an embodiment of this disclosure provides a lighting system, including:
a directional lighting apparatus, which includes a directional lighting drive controller and at least one directional lighting array, wherein a light emitted by the directional lighting array illuminates an area of a single patient support, directly or after being reflected by other medium;
a mounting framework, which is configured to fix the directional lighting array;
an input/output interface, which is configured to receive an instruction from a user;
a memory, which is configured to store configuration information of a default lighting mode and at least one customized lighting mode, wherein the configuration information of the default lighting mode includes multiple pieces of default color temperature configuration information which are associated with different working hours and/or multiple pieces of default illuminance configuration information which are associated with different working hours, wherein the configuration information of the customized lighting mode at least includes customized illuminance configuration information;
a processor, which is configured to control, when receiving a selection instruction for the at least one customized lighting mode from the user, the directional lighting apparatus to be switched from the default lighting mode, which is in operation, to a selected customized lighting mode, and to control, when a cancellation instruction is received from the user or the selected customized lighting mode is completed, the directional lighting apparatus to return to the default lighting mode.

A second aspect of an embodiment of this disclosure provides a medical suspension device, including:
a beam assembly, which is arranged above an area of a single patient support;
a suspension column, which is suspended below the beam assembly and is arranged at a side of the area of the single patient support, wherein the suspension column is configured to provide an electrical interface or installation device for configuration and use of a medical device which is used by a patient in the area of the single patient support; and
the lighting system described above, which is arranged at the beam assembly.

A third aspect of an embodiment of this disclosure provides a lighting system, including:
a long-time directional lighting apparatus, which includes a long-time directional lighting drive controller and at least one long-time directional lighting array, wherein a light emitted by the long-time directional lighting array illuminates an area of a single patient support, directly or after being reflected by other medium;
a short-time directional lighting apparatus, which includes a short-time directional lighting drive controller and at least one short-time directional lighting array, wherein a light emitted by the short-time directional lighting array illuminates the area of the single patient support, directly or after being reflected by other medium;
a mounting framework, which is configured to at least fix the long-time directional lighting array and the short-time directional lighting array;
an input/output interface, which is configured to receive an instruction from a user;
a memory, which is configured to store configuration information of a default lighting mode and at least one customized lighting mode, wherein the configuration information of the default lighting mode includes multiple pieces of default color temperature configuration information which are associated with different working hours and/or multiple pieces of default illuminance configuration information which are associated with different working hours, wherein the configuration information of the customized lighting mode at least includes customized illuminance configuration information;
a processor, which is configured to control the long-time directional lighting apparatus to execute the default lighting mode; and to control, if receiving a selection instruction for the at least one customized lighting mode from the user when the long-time directional lighting apparatus works in the default lighting mode, the short-time directional lighting apparatus to execute a selected customized lighting mode until a cancellation instruction is received from the user or the selected customized lighting mode is completed.

A fourth aspect of an embodiment of this disclosure provides a medical suspension device, including:
a beam assembly, which is arranged above an area of a single patient support;
a suspension column, which is suspended below the beam assembly and is arranged at a side of the area of the single patient support, wherein the suspension column is configured to provide an electrical interface or installation device for configuration and use of a medical device which is used by a patient in the area of the single patient support; and
the lighting system described above, which is arranged at the beam assembly.

A fifth aspect of an embodiment of this disclosure provides a lighting control method which is applied to a lighting system configured to provide lighting for an area of a single patient support; wherein the lighting system illuminates the area of the single patient support directly or after a light thereof being reflected by other medium, and includes a processor, a memory and a control panel; wherein the lighting control method includes:
powering on the lighting system;
invoking configuration information of a default lighting mode in the memory, wherein the configuration information of the default lighting mode comprises multiple pieces of default color temperature configuration information and multiple pieces of default illuminance configuration information; wherein the multiple pieces of default color temperature configuration information are associated with different working hours and the multiple pieces of default illuminance configuration information are associated with different working hours;
adjusting color temperature and illuminance of at least part of a directional lighting array in the lighting system according to the configuration information of the default lighting mode;
receiving an instruction from a user through the control panel;
determining a customized lighting mode according to the instruction from the user;
invoking configuration information of the customized lighting mode in the memory, wherein the configuration information of the customized lighting mode at least comprises customized illuminance configuration information; and
adjusting illuminance of at least part of the directional lighting array in the lighting system according to the configuration information of the customized lighting mode.

The lighting system, medical suspension device and lighting control method provided by embodiments of this disclosure, can work in the default lighting mode to dynamically adjust the color temperature or illuminance as time changes, so that the patient can perceive the changeable light similar to sunlight, and can also provide lighting through the instruction from the user to provide directional lighting to meet other requirements of the patient or the examination requirements of the medical staff. The same lighting system can provide at least two different modes of light for the patient at a single patient support to use without requiring configuration of other lighting modes, thus realizing the integration of lighting modes. Moreover, directional lighting can be set in the area of the single patient support, so that other patients are not affected.

### BRIEF DESCRIPTION OF THE DRAWINGS

FIG. 1 is a positional relationship diagram between a medical suspension device and a patient support in an embodiment of this disclosure.
FIG. 2 is a logic block diagram of a lighting system of an embodiment of this disclosure.
FIG. 3 is a logic block diagram of a lighting system in another embodiment of this disclosure.
FIG. 4 is a partial structural diagram of a lighting system of an embodiment of this disclosure.
FIG. 5 is a schematic diagram of another perspective of the structure shown in FIG. 4.
FIG. 6 is a schematic diagram of another perspective of the structure shown in FIG. 5.
FIG. 7 is the structural diagram of the structure shown in FIG. 4 omitting partial housing.
FIG. 8 is a schematic diagram of another perspective of the structure shown in FIG. 7.
FIG. 9 is a sectional view of the structure shown in FIG. 5, wherein the section plane is perpendicular to a longitudinal direction of a mounting framework.
FIG. 10 is a structural diagram of the mounting framework in FIG. 8.
FIG. 11 is a coordination diagram of the structure shown in FIG. 10 with a lighting array and an atmosphere lighting apparatus.
FIG. 12 is a partially enlarged schematic diagram of B in FIG. 9.
FIG. 13 is a partial structural diagram of a lighting system in another embodiment of this disclosure.
FIG. 14 is a circuit connection diagram of a first light source in a plurality of lighting arrays according to a first embodiment of this disclosure.
FIG. 15 is a circuit connection diagram of a first light source in a plurality of lighting arrays according to a second embodiment of this disclosure.
FIG. 16 is a circuit connection diagram of a first light source in a plurality of lighting arrays according to a third embodiment of this disclosure.
FIG. 17 is a circuit connection diagram of a first light source in a plurality of lighting arrays according to a fourth embodiment of this disclosure.
FIG. 18 is a structural diagram of a lens of a lighting array and a first light source which are installed together in an embodiment of this disclosure.
FIG. 19 shows rhythm change rule of color temperature and illuminance in a first rhythm mode of an embodiment of this disclosure.
FIG. 20 is a structural diagram of an input/output interface of an embodiment of this disclosure.
FIG. 21 is a schematic diagram of a display interface of an input/output interface of an embodiment of this disclosure.

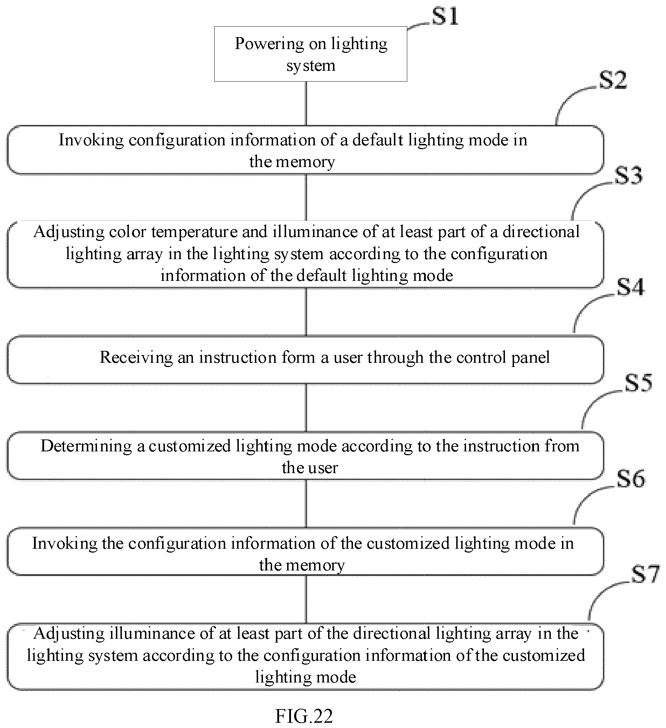

FIG. 22 is a flowchart of a lighting control method of a lighting system for a medical scene according to an embodiment of this disclosure.

### DESCRIPTION OF THE PREFERRED EMBODIMENTS

It should be noted that, in the case of no conflict, the embodiments and the technical features in the embodiments of this disclosure can be combined with each other. The detailed description in the specific implementation mode should be understood as an explanation purpose of this disclosure, and should not be regarded as an improper restriction on this disclosure.

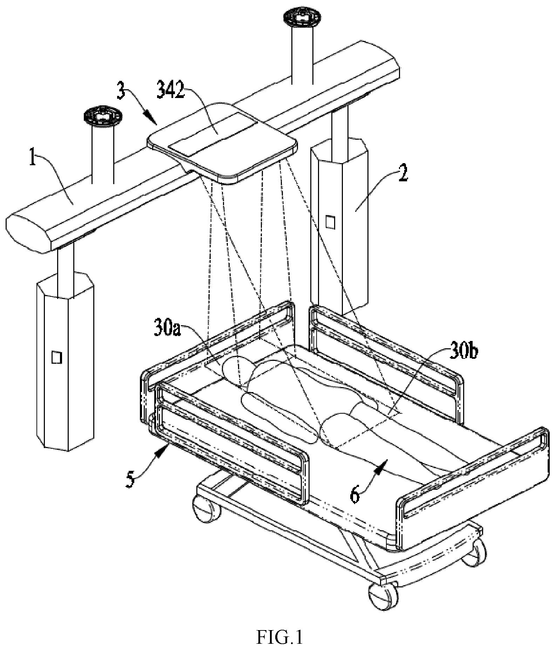

In the description of the embodiments of this disclosure, orientation or location relationship terms, such as "up", "down", "length direction", are based on the orientation or location relationship shown in FIG.1. It should be understood that these orientation terms are only for the convenience of describing this disclosure and simplifying the description, rather than indicating or implying that the device or element referred to must have a specific orientation, must be constructed and operated in a specific orientation, so they cannot be understood as restrictions on this disclosure.

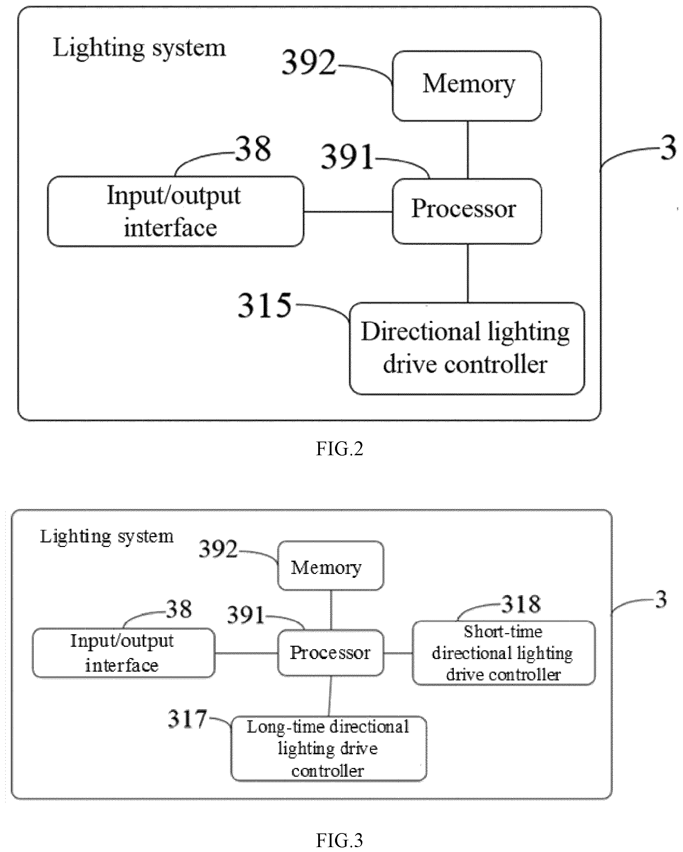

Referring to FIGS. 1, 2 and 7, one embodiment of this disclosure provides a lighting system 3 for providing lighting for an area of a single patient support, including a directional lighting apparatus, a mounting framework 35, an input/output interface 38, a memory 392, and a processor 391.

The directional lighting apparatus includes a directional lighting drive controller 315 and at least one directional lighting array 31a, wherein a light emitted by the directional lighting array 31a illuminates an area of a single patient support 5, directly or after being reflected by other medium. That is to say, the lighting light of the directional lighting array 31a of the lighting system is concentrated at a single patient support 5, which never interferes with other areas around the patient support 5. When there are multiple patient supports 5 in the same ward, the impact on patients 6 adjacent to the patient support 5 can be reduced.

The mounting framework 35 is configured to fix the directional lighting array 31a. That is, the mounting framework 35 at least provides a mounting position and mounting space for the directional lighting array 31a.

The memory 392 is configured to store configuration information of a default lighting mode and at least one customized lighting mode, wherein the configuration information of the default lighting mode includes multiple pieces of default color temperature configuration information and/or multiple pieces of default illuminance configuration information. The multiple pieces of default color temperature configuration information are associated with different working hours, and the multiple pieces of default illuminance configuration information are associated with different working hours, wherein the configuration information of the customized lighting mode at least includes customized illuminance configuration information. In one embodiment, the configuration information of the default lighting mode can only have multiple pieces of default color temperature configuration information, or only have multiple pieces of default illuminance configuration information, or have both multiple pieces of default color temperature configuration information and multiple pieces of default illuminance configuration information. In one embodiment, the working hours corresponding to the multiple pieces of default color temperature configuration information may not be associated with the working hours corresponding to the multiple pieces of default illuminance configuration information.

The processor is configured to control, when a selection instruction regarding the at least one customized lighting mode is received from the user, the directional lighting apparatus to be switched from the default lighting mode, which is in operation, to a selected customized lighting mode, and to control, when a cancellation instruction is received from the user or an execution is complete, the directional lighting apparatus to return to the default lighting mode. In the default lighting mode, the corresponding default color temperature configuration information and the corresponding default illuminance configuration information are invoked according to different working hours, and color temperature and illuminance of the directional lighting array 31a are adjusted through the directional lighting drive controller 315 according to the invoked default color temperature configuration information and the invoked default illuminance configuration information, so as to realize the dynamic adjustments of the color temperature and illuminance of the directional lighting array 31a. In the customized lighting mode, the corresponding configuration information is invoked and the illuminance of the directional lighting array 31a is adjusted through the directional lighting drive controller 315 according to the configuration information.

When the directional lighting apparatus works in the default lighting mode, the color temperature and illuminance of the directional lighting array 31a can be dynamically and regularly adjusted according to different working hours, that is, the default lighting mode is a rhythm mode, that is, the working state of the lighting system is the rhythm state by default, and the lighting system works in the rhythm state for a long time.

In some hospitals, ICU and other wards have no or few windows facing the natural outside, so the natural light cannot reach the position of patient 6, and the whole ward uses artificial lighting. In the wards with artificial lighting, the lighting lamps are turned on for a long time, the lighting environment in the whole room is constant, and there is no change in the lighting rhythm, which affects the rhythmic regulation function of patient 6, the secretion of melatonin of patient 6, and the sleep of patient 6 and medical staff, and may further be detrimental to the recovery and physical and mental health of patient 6, as well as the work and physical and mental health of medical staff. Therefore, the lighting system 3 of the embodiment of this disclosure can be used in the ICU to meet the rhythmic lighting of patients on a single patient support, which is conducive to the rehabilitation and physical and mental health of patient 6.

In addition, the lighting system of the embodiment of this disclosure provides customized lighting mode for user to select. Under different customized modes, the illuminance of the directional lighting array 31a is different. Therefore, user can select different customized lighting modes according to different illuminance requirements. For example, when patients need to read, the illuminance required is relatively small, and when medical staff need to examine the patient, the illuminance required is relatively large. Therefore, User can select the corresponding customized lighting mode according to their own needs. In the customized lighting mode, it mainly changes the illuminance of the lighting light without changing or absolutely not changing the color temperature of the lighting light. That is to say, the lighting system of the embodiment of this disclosure integrates rhythmic mode and a variety of customized lighting modes with high integration, which can meet a variety of lighting needs and is suitable for ICU and other wards.

In the embodiment of this disclosure, only the customized lighting mode or the default lighting mode can be selected, that is, the customized lighting mode and the default lighting mode cannot coexist, so the lighting under the customized lighting mode and the default lighting mode can be realized through the same directional lighting array 31a.

It needs to be noted that the light emitted by the directional lighting array 31a directly illuminates or is reflected by other medium to illuminates the area of the single patient support, includes the following three embodiments. The first is that the light emitted by the directional lighting array 31a directly illuminates the area of the single patient support. The second is that the light emitted by the directional lighting array 31a is reflected by other medium and then illuminates the area of the single patient support. The specific types of other medium are not limited, for example, they can be lenses, walls, mirrors, etc. The third is that there are many directional lighting arrays 31a and the light emitted by some directional lighting arrays 31a directly illuminates the area of the single patient support, while the light emitted by the other directional lighting arrays 31a illuminates the area of the single patient support after being reflected by other medium.

It should be noted that the processor can control, when a cancellation instruction is received from the user or an execution is complete, the directional lighting array 31a to return to the default lighting mode includes at least one of following ways.

In the first way, the processor 391 controls the directional lighting apparatus to return to the default lighting mode when a cancellation instruction is received from the user. That is, in this embodiment, the memory 392 does not store a preset execution period for the customized lighting mode. The working hours of the customized lighting mode depend on the actual requirement of the user. When the user needs to turn off the current customized lighting mode, the user inputs the cancellation instruction. Then the processor controls the directional lighting apparatus to return to the default lighting mode. In the second way, the processor 391 controls the directional lighting apparatus to return to the default lighting mode when the execution of the customized lighting mode is complete. In this embodiment, the memory 392 stores preset an execution period of the customized lighting mode. When the working hours of the customized lighting mode reaches the preset execution period, the processor 391 automatically controls the directional lighting apparatus to return to the default lighting mode.

In an embodiment, the directional lighting array 31a includes a plurality of first light sources 311 and a plurality of first lenses 312, wherein each first lens 312 is arranged correspondingly to at least one first light source 311. In each directional lighting array 31a, the light emitted by each first light source 311 converges in the convergence area, after being reflected by the first lens 312 corresponding to itself. The convergence of the light emitted by each first light source in the convergence area means that light spots which are formed by the lights in the convergence area can be at least partially overlapped, independent of each other without overlapped, or completely overlapped (this one has the best shadowless effect). The convergence area is located in an area of a single patient support. In this embodiment, each position in the convergence area can be illuminated by the light emitted by each first lens 312. When the medical staff examines the patient 6, not all the first lenses 312 are completely blocked by the head of the medical staff, and the light emitted by some of the first lenses 312 is not blocked. Therefore, the lights from these first lenses 312 can also be projected to the convergence area, in this way, there is basically no umbra area in the convergence area on the single patient support 5.

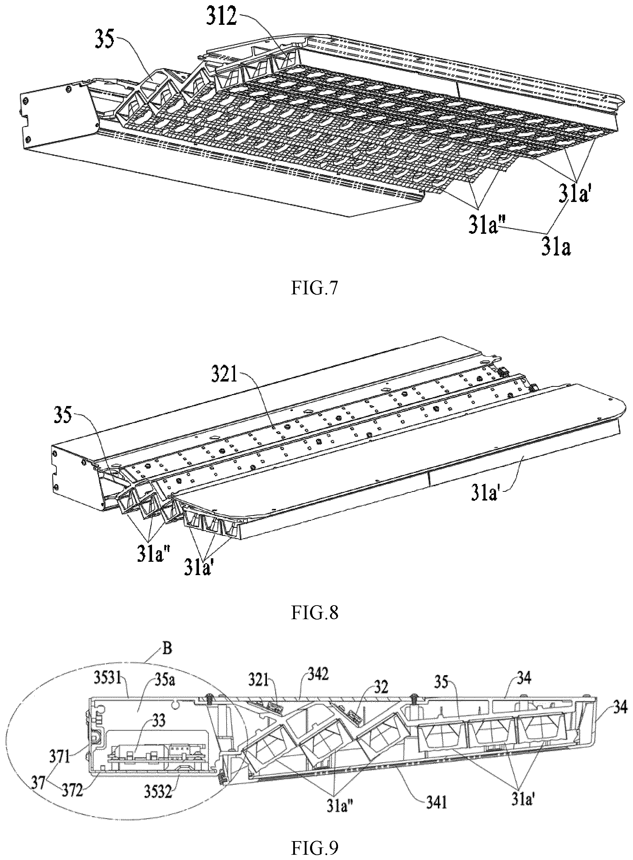

In one embodiment, there are a plurality of first lenses 312, such as eight first lenses 312, in each directional lighting array 31a. Referring to FIG. 7, the plurality of first lenses 312 in each directional lighting array 31a are arranged along a longitudinal straight line of the mounting framework 35.

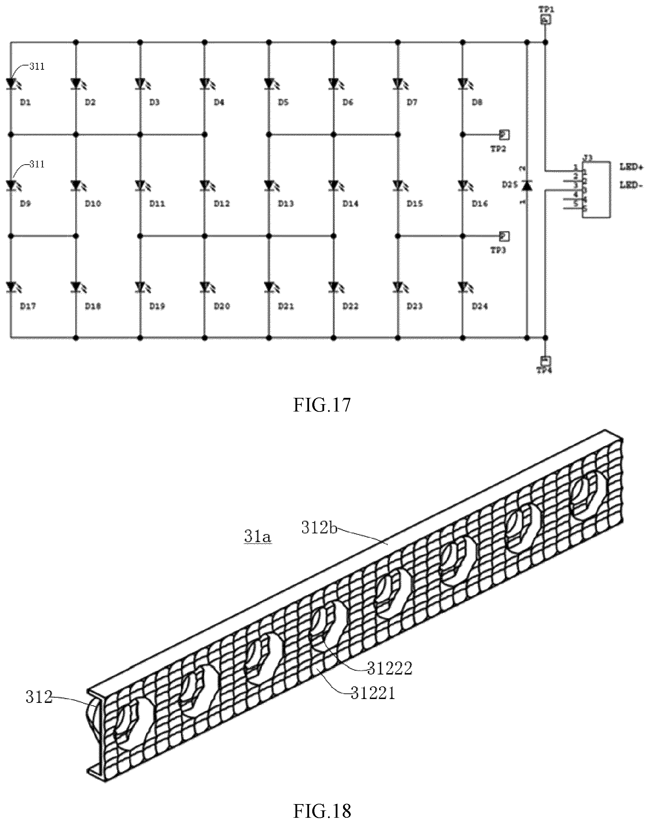

In one embodiment, please refer to FIG. 18, each directional lighting array 31a further includes a flange 312b at both lateral sides of an emergent surface 3122 of the first lens 312. The flange 312b extends along the arrangement direction of the plurality of first lenses 312, and each first lens 312 is located between the two flanges 312b. The flange 312b can improve the structural strength of the directional lighting array 31a.

In one embodiment, the first light source 311 corresponding to each first lens 312 includes a first cold white light source and a first warm white light source. That is to say, each first lens 312 is correspondingly provided with a plurality of first light sources 311. Among them, some of the first light sources 311 are the first cold white light sources, the light of the first cold white light source is cold white light, the others of the first light sources 311 are the first warm white light sources, and the light of the first warm white light source is warm white light. In this embodiment, the light emitted from each first lens 312 is the mixed light of the first cold white light source and the first warm white light source corresponding to the first lens 312. For example, the color temperature of the first warm white light source is 2200K, and the color temperature of the first cold white light source is 6500K.

In one embodiment, in each directional lighting array 31a, the first light source 311 corresponding to some of the first lenses 312 is the first cold white light source, and the first light source corresponding to the others of the first lenses 312 is the first warm white light source. That is, each first lens 312 corresponds to one first light source 311, and the color temperature and illuminance of the light emitted from each first lens 312 are the color temperature and illuminance of a single first light source 311. The color temperature and illuminance of light in the same directional lighting array 31a are the mixed color temperature and mixed illuminance of lights passing through all lenses 312 in the same directional lighting array 31a.

The color temperature and illuminance of the overall light of the directional lighting apparatus are the mixed color temperature and mixed illuminance of all the first light sources 311 which are turned on.

In one embodiment, the color temperature is adjusted by changing a turn-on ratio of the first light source 311 with different color temperatures. For example, the number of the turn-on first cold white light source is 12, and the number of the turn-on first warm white light source is 12. At this time, the ratio between the turn-on numbers of the first light sources 311 with two color temperatures is 1:1, which corresponds to one color temperature of the directional lighting array 31a. When it is necessary to adjust the color temperature, the turn-on number of the first cold white light source and the turn-on number of the first warm white light source can be adjusted. For example, the turn-on number of the first cold white light sources is 10, and the turn-on number of the first warm white light source is 12. At this time, the ratio between the turn-on numbers of the first light sources with two color temperatures is 5: 6. At this time, the color temperature corresponding to another color temperature of the directional lighting array 31a, that is, the color temperature of the directional lighting array 31a, has changed.

In another embodiment, the color temperature of the directional lighting apparatus can be adjusted by adjusting a photochromic ratio of the first light sources 311 corresponding to different color temperatures. Specifically, the photochromic ratio of the first light sources with different color temperatures is adjusted by changing currents of the first light sources corresponding to different color temperatures. For example, the current of the first cold white light source is 2mA, and the current of the first warm white light source is 3mA. This ratio corresponds to one color temperature of the directional lighting apparatus. When it is necessary to change the color temperature, for example, the current of the first cold white light source is adjusted to 3mA, and the current of the first warm white light source is adjusted to 4mA. At this time, the photochromic ratio of the first light sources corresponding to different color temperatures has changed, that is, the color temperature of the directional lighting apparatus has changed.

For example, the illuminance of the directional lighting apparatus is adjusted by changing the current corresponding to the first light sources 311 with different color temperatures in equal proportion, for example, the current of the first cold white light source is 2mA, the current of the first warm white light source is 3mA, and the current ratio of the first cold white light source and the first warm white light source is 2:3, which corresponds to one color temperature and one illuminance of the directional lighting apparatus. When it is only necessary to change the illuminance without adjusting the color temperature, the currents of the first cold white light source and the first warm white light source are increased or decreased in equal proportion. For example, the current of the first cold white light source is adjusted to 4mA, and the current of the first warm white light source is adjusted to 6mA. At this time, the illuminance can be adjusted while keeping the color temperature unchanged. It should be noted that when the currents of the first light sources 311 with different color temperatures varies in a non-equal proportion, both of the color temperature and illuminance of the directional lighting apparatus change.

In one embodiment, refer to FIG.7 and 8, the at least one directional lighting array 31a includes at least one first directional lighting array 31a' and at least one second directional lighting array 31a". That is, in this embodiment, there are multiple directional lighting arrays 31a, and some of the directional lighting arrays 31a are the first directional lighting array 31a', and the other directional lighting arrays 31a are the second directional lighting array 31a". The convergence area of the at least one first directional lighting array 31a' is a first convergence area 30a, that is, the convergence areas of all the first directional lighting arrays 31a' are the first convergence area 30a. A convergence area of the at least one second directional lighting array 31a "is the second convergence area 30b, that is, the convergence areas of all the second directional lighting arrays 31a" is the second convergence area 30b.

The first convergence area 30a and the second convergence area 30b respectively correspond to different body parts of the patient 6 lying in the area of the single patient support. For example, the first convergence area 30a corresponds to a head area of patient 6 on the patient support 5, and the second convergence area 30b corresponds to a trunk area of patient 6 on the patient support 5.

The processor 391 is further configured to independently control the first directional lighting array 31a or the second directional lighting array 31a" through the directional lighting drive controller 315 according to the instruction of the user. That is to say, the lighting for different body parts of the patient can be independently controlled, for example, the lighting of the head area and trunk area can be controlled separately to reduce the influence on the patient 6.

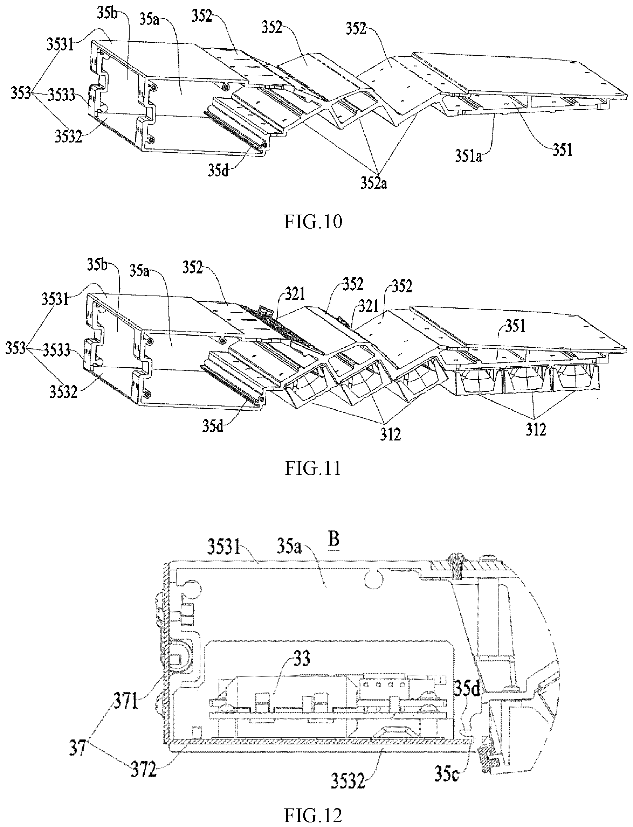

In one embodiment, refer to FIG. 11, the mounting framework 35 includes a first framework plate 351 and a second framework plate 352 connected with each other. The first framework plate 351 has a first mounting surface 351a, the first directional lighting array 31a' is mounted at the first mounting surface 351a of the first framework plate 351. The second framework plate 352 has a second mounting surface 352a, and the second directional lighting array 31a" is mounted at the second mounting surface 352a of the second framework plate 352. A normal of the first mounting surface 351a intersects with a normal of the second mounting surface 352a, and the intersection point is located inside a space between the lighting system and the area of the single patient support, that is, at least one of the first mounting surface 351a and the second mounting surface 352a inclines in the direction of the other. In this way, the first directional lighting array 31a' and the second directional lighting array 31a" have different inclination angles, such that the first directional lighting array 31a' and the second directional lighting array 31a" have different convergence areas.

In one embodiment, an acute angle formed between the first mounting surface 351a and the horizontal plane is smaller than an acute angle formed between the second mounting surface 352a and the horizontal plane, so that the light emitted by the second directional lighting array 31a" can be projected towards a farther area, for example, the light of the first directional lighting array 31a converges at the head area of the patient, while the light of the second directional lighting array 31a" converges at the trunk area of the patient, which is farther away.

In one embodiment, the lighting system further includes a housing 34, which is arranged around the mounting framework 35. The housing 34 forms an external surface of the lighting system 3, which can cover the structure which is arranged inside the mounting framework 35 and improve the appearance of the lighting system 3. The housing 34 further includes a first light transmission surface 341 arranged at a bottom side of the directional lighting array 31a. The light emitted by the directional lighting array 31a directly illuminates the area of the single patient support through the first light transmission surface 341, that is, the light emitted by the directional lighting array 31a leaves the lighting system after passing through the first light transmission surface 341. The light does not need to be reflected or transmitted again, and can directly reach the area of the single patient support. The distance between two endpoints of the first light transmission surface 341 is greater than or equal to 40 cm, that is, a linear distance between at least two endpoints of the first light transmission surface 341 is greater than 40 cm. This size is much larger than the head size of ordinary adults. When the medical staff examines the patient 6 on the patient support 5 under the lighting system 3, the first light transmission surface 341 is never completely blocked by the medical staff, and some lights passing through the first light transmission surface 341 can always reach the area of the patient support.

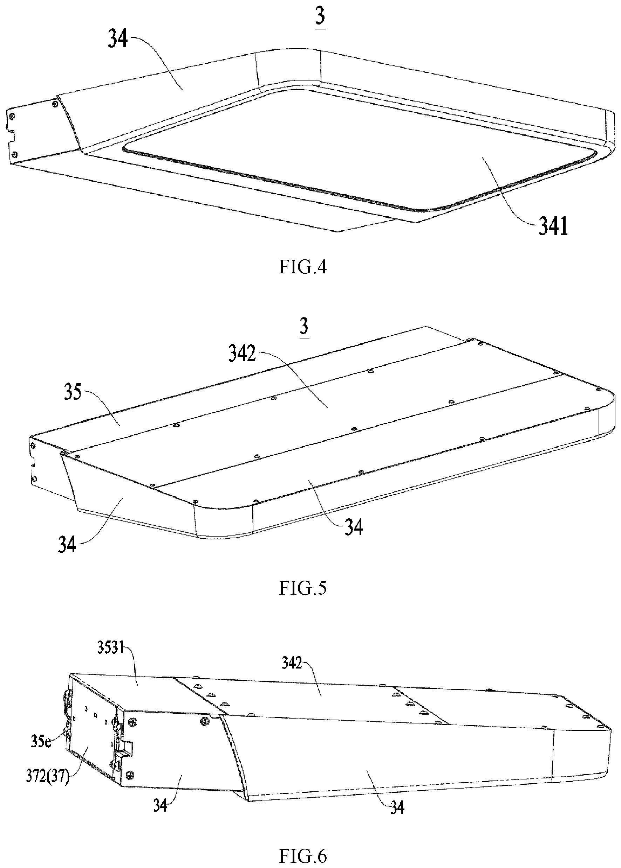

For example, in an embodiment, refer to FIG. 4, the first light transmission surface 341 is roughly rectangular, the width of the first light transmission surface 341 along the mounting framework 35 is greater than or equal to 30 cm, and the length of the first light transmission surface 341 along the mounting framework 35 is greater than or equal to 42 cm. That is, the area of the first light transmission surface 341 is greater than or equal to 1260 cm². It can be understood that the shape of the first light transmission surface 341 can also be other shapes, such as, ellipse, pentagon, etc.

It should be noted that the parts of the housing 34 that do not require light transmission are made of opaque materials. The materials of the housing 34 at the first light transmission surface 341 include but are not limited to the following materials: polymethylmethacrylate (commonly known as organic glass), polystyrene, polycarbonate, styrene acrylonitrile, etc.

In one embodiment, the lighting system further includes a housing 34, a main control board 33 and a power interface which are connected with each other. The housing 34 is arranged around the mounting framework 35. The housing 34 forms the external surface of the lighting system 3, which can cover the structure arranged inside the mounting framework 35, and improve the appearance of the lighting system 3. The housing 34 further includes a first light transmission surface 341 arranged at the bottom side of the directional lighting array 31a. The light emitted by the directional lighting array 31a directly illuminates the area of the single patient support through the first light transmission surface 341. The main control board 33 is arranged inside the housing 34, and the power interface is arranged at the housing 34. The lighting system of this embodiment can be used as an independent product, that is, the lighting system 3 is an independent packaging structure. When using, the user just need to plug the lighting system 3 in the power cord and power on the power interface.

It should be noted that in other embodiments, the lighting system 3 can also be used together with other products, for example, the lighting system 3 can be mounted on the wall, or integrated with a mechanical device. In the embodiment of this disclosure, the lighting system 3 is described as an example integrated with the medical suspension device.

In one embodiment, the configuration information of the default lighting mode includes a plurality of configuration information sets, and each configuration information set includes multiple pieces of default color temperature configuration information and multiple pieces of default illuminance configuration information. Where, under at least one color temperature working hour, the default color temperature configuration parameters corresponding to the color temperature working hour in at least two default color temperature configuration parameter sets are different, or under at least one illuminance working hour associated with the default illuminance configuration information, the default illuminance configuration parameters corresponding to the illuminance working hour in at least two default illuminance configuration parameter sets are different. The processor is configured to determine a configuration information set according to an instruction from the user, dynamically adjust the directional lighting array 31a, according to the multiple pieces of default color temperature configuration information in the determined configuration information set and the working hour which is associated with the multiple pieces of default color temperature configuration information, and according to the multiple pieces of default illuminance configuration information in the determined configuration information set and the working hour which is associated with the multiple pieces of default illuminance configuration information.

Specifically, for example, multiple pieces of default color temperature configuration information and multiple pieces of default illuminance configuration information in each configuration information set constitute a three-dimensional rhythm curve simulating time, illuminance and color temperature, that is, the illuminance and color temperature of the directional lighting array 31a change periodically according to the rhythm curve. For example, the illuminance and color temperature of the directional lighting array 31a change every day according to the above rhythm curve, that is, 24 hours of one day is a rhythm cycle.

Among them, multiple configuration information sets form multiple three-dimensional rhythm curves above, and the multiple rhythm curves never coincide completely, that is, the multiple rhythm curves can cross each other, but cannot completely coincide. At the same time, the multiple rhythm curves correspond to different color temperatures and/or illuminances.

When the lighting system works in the default lighting mode, supposing that the processor 391 controls the directional lighting drive controller 315 to execute information in the first configuration information set, that is, the color temperature and illuminance of the directional lighting array 31a change according to the first rhythm curve. After the user inputs a corresponding instruction, such as an instruction to change the illuminance or color temperature, the processor 391 determines another configuration information set corresponding to the instruction, that is, determines another corresponding rhythm curve according to the instruction of the user. Thereafter, the color temperature and illuminance of the directional lighting array 31a change according to the another rhythm curve.

In order to provide the choice of rhythmic illuminance or color temperature for the lighting system of this embodiment, users can adjust according to their actual needs to improve the user experience.

In one embodiment, the configuration information set is a set of color temperature configuration parameters and illuminance configuration parameters corresponding to the simulated circadian rhythm rules. The simulated circadian rhythm rules refer to the simulation of natural light conditions in nature 24 hours a day, which includes color temperature information and illuminance information, and the corresponding relationship among time, illuminance and color temperature, and is formed into a database and stored in memory 392.

In one embodiment, refer to FIG. 8. The at least one directional lighting array 31a includes a direct directional lighting array and a reflective directional lighting array 321, wherein a light emitted by the direct directional lighting array directly illuminates the area of the single patient support, and a light emitted by the reflective directional lighting array 321 illuminates the area of the single patient support after being reflected through a ceiling of the area of the single patient support. That is to say, in this embodiment, the reflective directional lighting array 321 projects light towards the ceiling, and then emits the reflect light from the ceiling to the area of the single patient support.

In one embodiment, refer to FIG. 5 and 6. The housing 34 further includes a second light transmission surface 342 arranged above the reflective directional lighting array 321. The light emitted by the reflective directional lighting array 321 illuminates the ceiling after being projected by the second light transmission surface 342.

In one embodiment, the customized lighting mode includes at least one of reading mode, examination mode and light stimulation mode.

In the embodiment where the customized lighting mode includes the reading mode, the processor 391 is configured to adjust the illuminance of the directional lighting array 31a in the range of 300lx-2000lx and adjust the color temperature of the directional lighting array 31a in the range of 3000K-5000K and keep the color temperature unchanged, through the directional lighting drive controller 315, in the reading mode. For example, when the patient 6 needs to read on the patient support 5, high illuminance lighting is not required at this time. The above color temperature range and illuminance range can better meet the lighting needs of patient 6 when reading. It should be noted that in the reading mode, the color temperature of the directional lighting array 31a is unchanged, and the illuminance is not limited. That is, in some embodiments, the color temperature cannot be adjusted, but the illuminance can be adjusted. In another embodiment, both the illuminance and color temperature cannot be adjusted.

In the embodiment where the customized lighting mode includes the examination mode, the processor 391 is configured to adjust the illuminance of the directional lighting array 31a in the 1000lx-10000lx range and adjust the color temperature in the range of 3000K-5000K and keep the color temperature unchanged, through the directional lighting drive controller 315, in the examination mode. For example, when the medical staff needs to examine the patient 6, and the above color temperature and illuminance ranges are required in the trunk area of the patient 6, and under which color temperature and illuminance ranges the medical staff can perform clinical operations such as intubation. It should be noted that in the examination mode, the color temperature of the directional lighting array 31a is unchanged, and the illuminance is not limited. That is, in some embodiments, the color temperature cannot be adjusted, but the illuminance can be adjusted. In another embodiment, both the illuminance and color temperature cannot be adjusted.

In the embodiment where the customized lighting mode includes the light stimulation mode, the processor 391 is configured to adjust the illuminance of the directional lighting array 31a to gradually change with the highest value of the illuminance in the range of 2000 lx-10000 lx and keep the color temperature unchanged, through the directional lighting drive controller 315, in the light stimulation mode. It can be understood that in the light stimulation mode, the light of the directional lighting array 31a can at least project onto the head area of the patient, so that the eyes of the patient can feel the light. It should be noted that under the light stimulation mode, the color temperature of the directional lighting array 31a is unchanged, and the illuminance is not limited. That is, in some embodiments, the color temperature cannot be adjusted, but the illuminance can be adjusted. In another embodiment, both the illuminance and color temperature cannot be adjusted.

For example, when it is necessary to wake up a sleeping patient 6, some of the first light sources 311 provide a time-controllable regional lighting with high illuminance for the head area of the patient 6. The eyes of the patient can feel the lighting of high illuminance to wake up. For example, in one embodiment, in the light stimulation mode, the illuminance of the head area of patient 66 gradually changes within a preset time period, for example, the sum of the preset light stimulation time periods is 10 minutes to 20 minutes, and the illuminance gradually increases from no light to about 2000 lx to 10000 lx, and then gradually extinguishes.

In some embodiments, the lighting system 3 includes a main control board 33, which is electrically connected with the directional lighting drive controller 315.

Refer to FIG. 9 to 12. In one embodiment, the lighting array 31a is arranged at a first end of the mounting framework 35 in a transverse direction, while a second end of the mounting framework 35 in the transverse direction is provided with an accommodation cavity 35a, and the main control board 33 is arranged inside the accommodation cavity 35a. In this way, the main control board 33 can be limited inside the mounting framework 35, so as to prevent the main control board 33 from protruding from the outer surface of the mounting framework 35 and being rubbed by other parts. In addition, the main control board 33 is arranged at the second end of the mounting framework 35 in the transverse direction, which can also avoid interference between the mounting position of the main control board 33 and the mounting positions of the lighting array 31a and the second light source 321. It should be noted that the specific shape of the accommodation cavity 35a is not limited, as long as it can accommodate the main control board 33.

In one embodiment, the mounting framework 35 is a metal piece, that is, the material of the mounting framework 35 is metal. By using the thermal conductivity of the metal, the heat generated by the heating elements in the lighting system 3 can be dissipated in time. For example, the heat generated by the main control board 33 and the light source can be dissipated in time. The specific types of metal materials for mounting the framework 35 are not limited, as long as they can conduct heat, for example, copper, iron, aluminum, etc. The metal materials can be pure metal or alloy. The mounting framework 35 can be made of the same metal for the whole structure, or different metals for different parts. There is no restriction here.

In order to avoid direct contact between the main control board 33 and the metal mounting framework 35, in an embodiment, refer to FIG.12, the lighting system 3 includes an insulating support 37, the main control board 33 is fixed at the insulating support 37, and the insulating support 37 is fixedly connected with the mounting framework 35. In other words, the main control board 33 and the mounting framework 35 are separated by the insulating support 37 to ensure electrical insulation between the main control board 33 and the mounting framework 35 and prevent conductive contact.

In one embodiment, refer to FIG. 10 and 11. An opening 35b is arranged at a side of the accommodation cavity 35a away from the first end. A part of the structure of the insulating support 37 can be disposed into the accommodation cavity 35a from the opening 35b. Refer to FIG.12. Another part of the structure of the insulating support 37 is located outside the accommodation cavity 35a and is fixedly connected with the periphery of the opening 35b. During the assembly process, the main control board 33 can be fixed at the insulating support 37, and then the insulating support 37 can be pushed from the opening 35b to the accommodation cavity 35a until a part of the insulating support 37 abuts against the periphery of the opening 35b, so that the fast positioning of the insulating support 37 can be achieved. Then, the insulating support 37 can be fixedly connected with the periphery of the opening 35b outside the mounting framework 35. It can be seen that there is a large operation space during the assembly process, which is convenient for assembly. In this embodiment, after the insulating support 37 is placed inside the accommodation cavity 35a, it is unnecessary to fasten and connect the parts of the insulating support 37 which are located inside the accommodation cavity 35a.

In order to prevent the insulating support 37 located inside the accommodation cavity 35a from deflecting, in one embodiment, refer to FIG.12, the accommodation cavity 35a is provided with a clamping groove 35c, and an end of the insulating support 37 which is away from the opening 35b is clamped into the clamping groove 35c. The end of the insulating support 37 which is away from the opening 35b is limited by the clamping groove 35c, so that the end of the insulating support 37 which is away from the opening 35b never deflects, and the stability of the insulating support 37 is increased.

The specific structure of the clamping groove 35c is adapted to an end shape of the insulating support 37. In one embodiment, referring to FIG. 11 and 12, a wall surface of the accommodation cavity 35a is provided with a convex rib 35d, an extension direction of the convex rib 35d is the same as an extension direction of the end of the insulating support 37, and the above clamping groove 35c is formed between the convex rib 35d and a bottom surface of an accommodation space 3125. When the end of the insulating support 37 is inserted into the clamping groove 35c, the convex rib 35d stops and limits the insulating support 37 to prevent the end of the insulating support 37 from deflecting towards the convex rib 35d.

The specific structure of insulating support 37 is not limited. For example, in an embodiment, refer to FIG.12, the insulating support 37 is roughly L-shaped. Specifically, the insulating support 37 includes a first insulating plate 371 and a second insulating plate 372 that are connected with each other. The first insulating plate 371 is located outside the accommodation cavity 35a and abuts against the periphery of the opening 35b, the second insulating plate 372 is located inside the accommodation cavity 35a, and the main control board 33 is located at the second insulating plate 372. The second insulating plate 372 is supported at the bottom surface of the accommodation space 3125. The end of the second insulating plate 372 which is away from the first insulating plate 371 is clamped into the above clamping groove 35c. In this embodiment, the insulating support 37 and the mounting framework 35 are matched in a similar way to a drawer.

In one embodiment, refer to FIG.6, the second insulating plate 372 covers the opening 35b, that is, the opening 35b is closed by the second insulating plate 372. The second insulating plate 372 can play the role of fixed mounting on the one hand and covering the opening 35b on the other hand. In this embodiment, the second insulating plate 372 forms the external surface of the lighting system 3.

In one embodiment, in order to facilitate the mounting of the lighting system 3 at other devices or walls, refer to FIG. 6, a mounting structure 35e is arranged at the mounting framework 35, and the lighting system 3 is mounted to other structures through the mounting structure 35e. In other words, a connection structure for external connection is reserved at the lighting system 3. When the lighting system 3 does not need to be integrated into other devices or walls, the lighting system 3 can be used alone. When it is necessary to integrate the lighting system 3 into other devices or walls for use, the lighting system 3 can be mounted through the mounting structure 35e, which can increase the usage selectivity of the lighting system 3.

The specific structure of the mounting structure 35e is not limited, for example, it can be a hook, a clip or the like structure. In one embodiment, the mounting structure 35e is a threaded hole. During mounting, only need to pass screws through the connected body and the above threaded holes from the outside, and the mounting method is simple and reliable.

The mounting structure 35e is arranged around the opening 35b, so that when the lighting system 3 is used, the user never sees the second insulating plate 372 and the opening 35b, so as to prevent the second insulating plate 372 and the opening 35b from affecting the appearance of the lighting system 3.

In one embodiment, refer to FIG. 10 and 11. The mounting framework 35 includes a frame body 353. One end of the frame body 353 is connected with the first framework plate 351. The second framework plate 352 is connected between the first framework plate 351 and the frame body 353. The other end of the frame body 353 is formed with the above-mentioned opening 35b. The space inside the frame body 353 is the accommodation cavity 35a. Specifically, the frame body 353 includes a top plate 3531, a bottom plate 3532, and two connecting plates 3533 connected between the top plate 3531 and the bottom plate 3532. The two connecting plates 3533 are located at opposite sides of the opening 35b. The top plate 3531, the bottom plate 3532, and the two connecting plates 3533 together enclose the opening 35b.

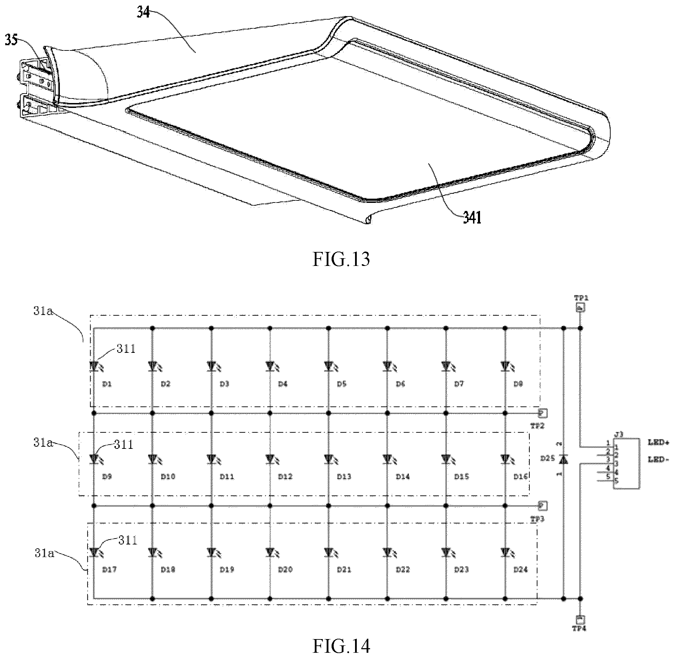

In one embodiment, refer to FIG. 11 and 13. The mounting framework 35 is open at opposite ends along the extension direction of the clamping groove 35c. During the assembly process, the operator can assemble from the open position of the mounting framework 35. Refer to FIG.11. In some embodiments, after the assembly is completed, the housing 34 is used to close the open position of the mounting framework 35. In this way, electrical elements such as the main control board 33 can be completely enclosed inside the lighting system 3, so that there are no exposed electrical elements outside the lighting system 3, and the safety of the lighting system 3 is improved. Refer to FIG.13. In other embodiments, the opening of the mounting framework 35 does not need to be closed. During installation, the mounting framework 35 can be embedded into other structures for shielding.

In some embodiments, at least some of the plurality of first light sources 311 of the directional lighting array 31a are arranged in parallel in the circuit, that is, not all the first light sources 311 are connected in series, and the connection circuit of the first light source 311 is divided into a plurality of parallel branches. If the first light source 311 in one of the parallel branches fails, the first light source 311 in the other parallel branches can be used normally, without causing the entire directional lighting array to be scrapped.

The parallel connection mode of the plurality of first light sources 311 in the circuit is not limited. For example, please refer to FIG.14. In the circuit connection diagram of the first light source 311 according to a first embodiment of this disclosure, the first light source 311 of the directional lighting array adopts a matrix connection method. All the first light sources 311 in the same lighting array 31a are connected in parallel, and the plurality of lighting arrays 31a are connected in series. Specifically, for example, the first light source 311 is an LED (Light Emitting Diode) and there are three lighting arrays 31a each has eight LEDs. When one LED in one of the three lighting arrays 31a fails, the current can flow through the remaining seven branches, that is, the LED in the remaining seven branches can still be used normally. It should be noted that in other embodiments, the number of the LEDs in each lighting array 31a can be equal or not. The LEDs in each lighting array 31a can be partially connected in series and partially connected in parallel.

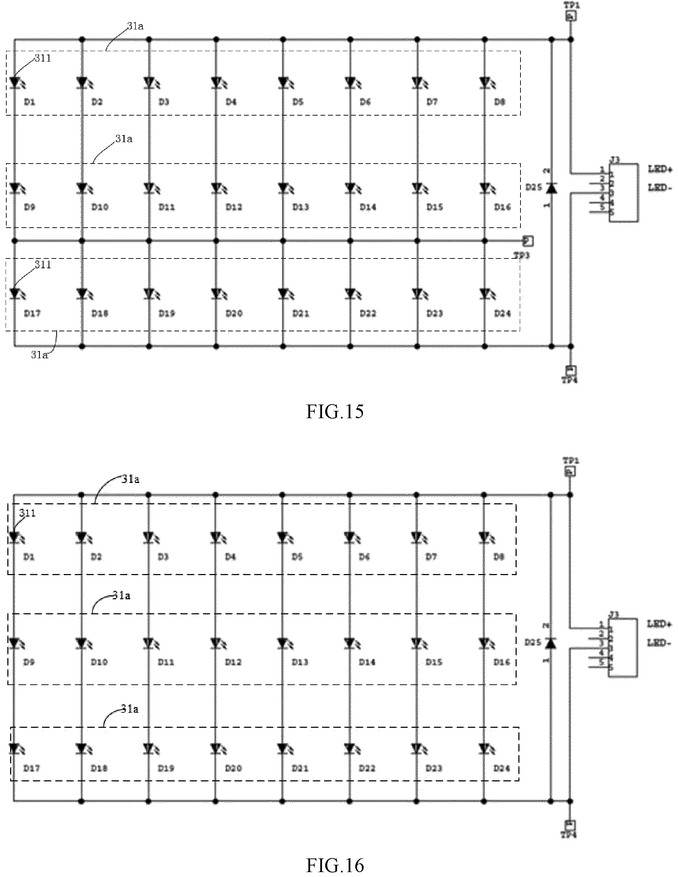

Refer to FIG.15, which is a circuit connection diagram of a plurality of first light source 311 according to a second embodiment of this disclosure. In this embodiment, similarly, for example, the first light source 311 is an LED (Light Emitting Diode) and there are three lighting arrays 31a each one has eight LEDs. Eight LEDs in each first lighting array 31a are arranged in eight parallel branches, and the branches in two lighting arrays 31a are in one-one correspondence and arranged in series, so that two lighting arrays 31a form eight parallel branches together and then they are connected in series with another lighting array 31a. Among them, each parallel branch is provided with two LEDs.

Refer to FIG.16, which is a circuit connection diagram of a plurality of first light source 311 according to a third embodiment of this disclosure. In this embodiment, similarly, for example, the first light source 311 is an LED (Light Emitting Diode) and there are three lighting arrays 31a each one has eight LEDs. Each first lighting array 31a has eight branches, and the branches in the three lighting arrays 31a are in one-one correspondence and arranged in series, so that the three lighting arrays 31a together form eight parallel branches, and each parallel branch is provided with three LEDs.

Please refer to FIG. 17, which is a circuit connection diagram of a plurality of first light source 311 according to a fourth embodiment of this disclosure. In this embodiment, the circuits of LEDs of three lighting arrays are in hybrid arrangement.

It can be understood that the circuit connection mode of the plurality of first light sources 311 is not limited. In addition to the above implementation, there can be other embodiments. For example, all first light sources 311 can be arranged in parallel, that is, just one first light source 311 is arranged in each parallel branch.

Similarly, at least some of the plurality of second light sources 321 are arranged in parallel in the circuit, that is, a circuit of the atmosphere lighting apparatus is divided into a plurality of parallel branches. In this way, when the second light source 321 in one of the parallel branches fails, the second light source 321 in the other parallel branches can be used normally, without causing the entire atmosphere lighting apparatus to be scrapped.

It should be noted that the second light source 321 can adopt the circuit connection mode of each embodiment of the first light source 311 described above. Of course, the second light source 321 can also adopt other circuit connection modes, which is not repeated here.

Another embodiment of this disclosure provides a medical suspension device. Please refer to FIG.1, the medical suspension device includes a beam assembly 1, a suspension column 2, and a lighting system 3 in any of the above embodiments. Wherein, the beam assembly 1 is arranged in the area of the single patient support. The suspension column 2 is suspended below the beam assembly 1 and is arranged at a side of the area of the single patient support. The suspension column 2 is configured to provide an electrical interface or installation device for configuration and use of a medical device (such as, a ventilator, monitor and other medical device) which is used by the patient 6 in the area of the single patient support. Wherein, electrical interfaces, signal line interfaces, power supply interface, gas source interface, etc., as well as, electrical wires, gas pipelines, electrical components, etc. of medical suspension device are arranged inside the suspension column 2.

In some embodiments, a first end of the mounting framework 35 protrudes from a lateral side of the beam assembly 1 in the direction of the area of the single patient support, and a second end of the mounting framework 35 is fixedly connected with the beam assembly 1.

The beam assembly 1 has a large mounting space to mount the lighting system 3. In addition, the beam assembly 1 has a large height relative to the ground, and the mounting of the lighting system 3 at the beam assembly 1 never occupies activity space of the medical staff or placement space of other devices. Furthermore, it is possible to enable the light of the lighting system 3 to have a suitable propagation distance.

There is no limit to the number of the suspension columns 2, which can be one, two or more. In the embodiment of this disclosure, two suspension columns 2 are taken as an example to describe. A patient support 5 is placed in front of the beam assembly 1 and between two suspension columns 2 which are used by the patient support 5. It can be understood that in other embodiments, the same one medical suspension device can be simultaneously used by the two patient supports 5 which are in the front and back respectively. In this embodiment, two above-mentioned lighting systems 3 can be arranged at the beam assembly 1, and each lighting system 3 is used for one corresponding patient support 5.

In one embodiment, please continue to refer to FIG.1. The patient support 5 is placed at the middle position of the ground along the length direction of the beam assembly 1. The lighting system 3 is roughly located at the middle position of the beam assembly along the length direction of the beam assembly. In this way, the lighting system 3 can be aligned with the corresponding patient support 5.

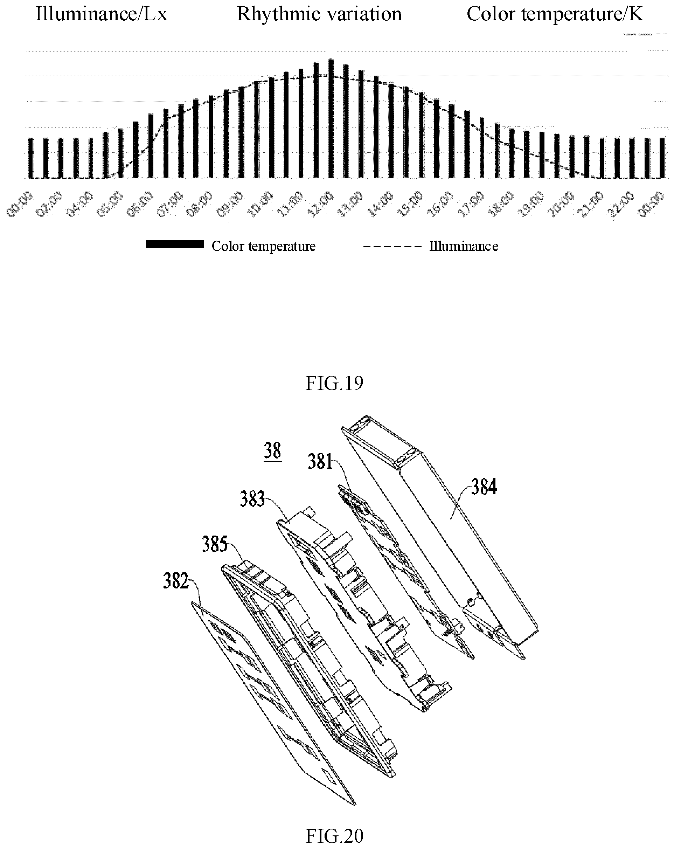

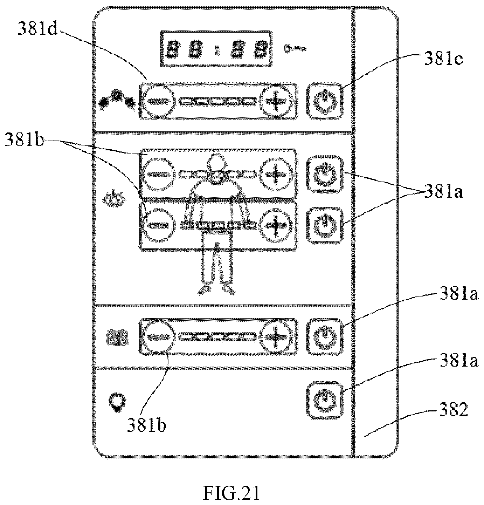

In one embodiment, refer to FIG.20. The input/output interface 38 includes a display controller 381 and a display panel 382 which are connected with each other. Refer to FIG.21, wherein the display panel 381 is provided with a switch control element 381a and an adjustment control element 381b for the customized lighting mode, and an adjustment control element 381d for the default lighting mode. The adjustment control element 381b for the customized lighting mode is configured to adjust the illuminance, and the adjustment control element 381d for the default lighting mode is configured to adjust the illuminance and/or color temperature. The processor 381 is further configured to determine an instruction from a user through control element information of the display panel which is selected by the user and identified by the display controller 381. When the directional lighting apparatus works in the default lighting mode, it does not respond to the instruction from the user of the adjustment control element 381b for the customized lighting mode. When the directional lighting apparatus works in the customized lighting mode, it does not respond to the instruction from the user of the adjustment control element 381d for the default lighting mode.

In this embodiment, the processor 391 responds to the instruction from the user only if the lighting mode corresponding to the adjustment control element triggered by the user is the same as the current lighting mode of the directional lighting apparatus. Specifically, when the directional lighting apparatus works in the customized lighting mode, if the user triggers the adjustment control element 381d corresponding to the default lighting mode, the processor 391 does not respond to the instruction from the user, that is, at this time, the instruction from the user is invalid. Similarly, when the directional lighting apparatus works in the default lighting mode, if the user triggers the adjustment control element 381b corresponding to the customized lighting mode, the processor 391 does not respond to the instruction from the user, that is, at this time, the instruction from the user is invalid.

In a specific embodiment, the adjustment control element on the display panel 382 can be mechanical keys, as well as, virtual buttons, touch keys arranged on the display screen, etc., which are not limited here.

It should be noted that, in an embodiment, please refer to FIG.21 again. The display panel 382 is further provided with a switch control element 381c corresponding to the default lighting mode, and the display panel 382 is provided with a switch control element 381a and an adjustment control element 381b corresponding to each customized lighting mode, that is, each customized lighting mode corresponds to one switch control element 381a and one adjustment control element 381b.

In one embodiment, refer to FIG.20. The input/output interface 38 further includes a rear housing 384 and a front housing 383. The display panel 382 is arranged at a front side of the front housing 383. For example, the display panel 382 can be bonded to the front side of the front housing 383 through double-sided adhesive tape. The display controller 381 is arranged at a rear side of the front housing 383. For example, the display controller 381 can be bonded to the rear side of the front housing 383 through double-sided adhesive tape. The display panel 382, the front housing 383 and the rear housing 384 form an enclosed space together, and the display controller 381 is arranged inside the enclosed space between the front housing 383 and the rear housing 384. In this way, electric elements can be enclosed in the enclosed space. Even if the input/output interface 38 is not attached to other mechanical device, the input/output interface 38 can also be used as a safe module for user to operate directly without electric shock risk.

In one embodiment, please refer to FIG.20. The input/output interface 38 further includes a key fixing frame 385, which is fixedly connected with the front housing 383 or the rear housing 384. The input/output interface 38 is connected with mechanical device through the key fixing frame 385. For example, the key fixing frame 385 can be provided with a connecting hole, which can be detachably connected by connecting pieces such as screws or bolts.

It can be understood that, in order to make the mounting of the input/output interface 38 more reliable, the key fixing frame 385 surrounds circumference of the front housing 383 or the rear housing 384, and there are plurality of connecting holes which are arranged at intervals along an edge of the key fixing frame 385. In this way, the circumferential stress of the key fixing frame along the circumference of the front housing 383 or the rear housing 384 is relatively uniform, and the mounting reliability of the input/output interface 38 is improved.

In one embodiment, the input/output interface 38 is arranged at the suspension column 2 to improve the integration of the medical suspension device, and facilitate the information interaction between the medical staff and the input/output interface 38, as well as the operation of the medical staff.

In one embodiment, the lighting system 3 further includes a main control board 33. The directional lighting array 31a is connected with the directional lighting drive controller 315 at the main control board 33 through a cable. The main control board 33 is arranged inside the beam assembly 1, and one end of the cable passes through the mounting framework 35 and extends into the beam assembly 1. In this embodiment, the main control board 33 is arranged inside the beam assembly 1, and the beam assembly 1 has a large space to facilitate the heat dissipation of the main control board 33.

In another embodiment, the main control board 33 is arranged inside the mounting framework 35. It should be noted that the structure for mounting the main control board 33 at the mounting framework 35 can refer to the structure in the first embodiment, and is not repeated here for concise.

Another embodiment of this disclosure provides a lighting system for providing lighting for an area of a single patient support, including a long-time directional lighting apparatus, a short-time directional lighting apparatus, a mounting framework 35, an input/output interface 38, a memory 392, and a processor 391. The long-time directional lighting apparatus includes a long-time directional lighting drive controller 317 and at least one long-time directional lighting array. A light emitted by the long-time directional lighting array illuminates the area of the single patient support, directly or after being reflected by other medium. The short-time directional lighting apparatus includes a short-time directional lighting drive controller 318 and at least one short-time directional lighting array. A light emitted by the short-time directional lighting array illuminates the area of the single patient support, directly or after being reflected by other medium. That is to say, lighting lights of the long-time directional lighting array and the short-time directional lighting array are concentrated at one single patient support 5, which never interfere with other areas around the patient support 5. If there are the plurality of patient supports 5 in the same ward, the influence on patients 6 of adjacent patient supports 5 can be reduced.

The mounting framework is configured to fix the long-time directional lighting array and short-time directional lighting array at least. The input/output interface is configured to receive an instruction from a user.

The memory is configured to store configuration information of a default lighting mode and at least one customized lighting mode, wherein the configuration information of the default lighting mode includes multiple pieces of default color temperature configuration information which are associated with different working hours and/or multiple pieces of default illuminance configuration information which are associated with different working hours, wherein the configuration information of the customized lighting mode at least includes customized illuminance configuration information. In one embodiment, the configuration information of the default lighting mode just includes multiple pieces of default color temperature configuration information, or just includes multiple pieces of default illuminance configuration information. Optionally, the configuration information of the default lighting mode can further include multiple pieces of default color temperature configuration information and multiple pieces of default illuminance configuration information. In one embodiment, the working hours corresponding to the multiple pieces of default color temperature configuration information may not be associated with the working hours corresponding to the multiple pieces of default illuminance configuration information.

The processor is configured to control the long-time directional lighting apparatus to execute the default lighting mode; and to control, if a selection instruction regarding the at least one customized lighting mode is received from the user when the long-time directional lighting apparatus works in the default lighting mode, the short-time directional lighting apparatus to execute a selected customized lighting mode until a cancellation instruction is received or the execution is complete. In the default lighting mode, the corresponding default color temperature configuration information and the corresponding default illuminance configuration information are invoked according to different working hours, and the color temperature and illuminance of the long-time directional lighting array are adjusted through the long-time directional lighting drive controller according to the invoked default color temperature configuration information and the invoked default illuminance configuration information, so as to realize the dynamic adjustments of the color temperature and illuminance of the long-time directional lighting array. In the customized lighting mode, the corresponding configuration information is invoked and the illuminance of the short-time directional lighting array is adjusted through the short-time directional lighting drive controller according to the configuration information.

In the lighting system of this embodiment, the default lighting mode is realized through the long-time directional lighting array, and the customized lighting mode is realized through the short-time directional lighting array. In the default lighting mode of the lighting system in the embodiment of this disclosure, the color temperature and illuminance of the long-time directional lighting array can be dynamically and regularly adjusted according to different working hours, that is, the default lighting mode is the rhythmic mode, that is, the default lighting mode is a rhythm mode. That is, the working state of the lighting system is the rhythm state by default, and the lighting system works in the rhythm state for a long time.

In some hospitals, ICU and other wards have no or few windows facing the natural outside, so the natural light cannot reach the position of patient 6, and the whole ward uses artificial lighting. In the wards with artificial lighting, the lighting lamps are turned on for a long time, the lighting environment in the whole room is constant, and there is no change in the lighting rhythm, which affects the rhythmic regulation function of patient 6, the secretion of melatonin of patient 6, and the sleep of patient 6 and medical staff, and may further be detrimental to the recovery and physical and mental health of patient 6, as well as the work and physical and mental health of medical staff. Therefore, the lighting system 3 of the embodiment of this disclosure can be used in the ICU to meet the rhythmic lighting of patients on a single patient support, which is conducive to the rehabilitation and physical and mental health of patient 6.

In addition, the lighting system of the embodiment of this disclosure provides customized lighting mode for user to select. Under different customized modes, the illuminance of the short-time directional lighting array is different. Therefore, user can select different customized lighting modes according to different illuminance requirements. For example, when patients need to read, the illuminance required is relatively small, and when medical staff need to examine the patient, the illuminance required is relatively large. Therefore, User can select the corresponding customized lighting mode according to their own needs. In the customized lighting mode, it mainly changes the illuminance of the lighting light without changing or absolutely not changing the color temperature of the lighting light.

That is to say, the lighting system of the embodiment of this disclosure integrates rhythmic mode and a variety of customized lighting modes with high integration, which can meet a variety of lighting needs and is suitable for ICU and other wards.

In the embodiment of this disclosure, the customized lighting mode and the default lighting mode can exist at the same time, that is, at the same time, both the long-time directional lighting array and the short-time directional lighting array can project light into the area of the single patient support. It should be noted that the light emitted by the long-time directional lighting array illuminates the area of the single patient support, directly or after being reflected by other medium, includes the following three embodiments. The first is that the light emitted by the long-time directional lighting array directly illuminates the area of the single patient support. The second is that the light emitted by the long-time directional lighting array is reflected by other medium and then illuminates the area of the single patient support. The specific types of other medium are not limited, for example, they can be lenses, walls, mirrors, etc. The third is that there are many long-time directional lighting arrays and the light emitted by some long-time directional lighting arrays directly illuminates the area of the single patient support, while the light emitted by the other long-time directional lighting arrays illuminates the area of the single patient support after being reflected by other medium. The light emitted by the short-time directional lighting array illuminates the area of the single patient support, directly or after being reflected by other medium, includes the similar above three embodiments of the long-time directional lighting array.

It should be noted that controlling the short-time directional lighting array to execute the selected customized lighting mode until the cancellation instruction is received or the execution is complete, can include two following embodiments. In the first embodiment, the processor can control, when the cancellation instruction is received from the user, the short-time directional lighting array to stop executing the selected customized lighting mode. That is, in this embodiment, the memory does not store a preset execution period for the customized lighting mode. The working hours of the customized lighting mode depend on the actual requirement of the user. When the user needs to turn off the current customized lighting mode, the user inputs the cancellation instruction. Then the processor controls the short-time directional lighting array to stop emitting light. In the second embodiment, the processor controls the short-time directional lighting array to stop executing the selected customized lighting mode, when the selected customized lighting mode is completed. That is, in this embodiment, the memory stores a preset execution period for the customized lighting mode. When the working hours of the customized lighting mode reaches the preset execution period, the processor automatically controls the short-time directional lighting array to stop emitting light.

In an embodiment, the light emitted by the short-time directional lighting array illuminates the area of the single patient support directly. The short-time directional lighting array includes a plurality of second light sources and a plurality of second lenses, wherein each second lens is correspondingly provided with at least one second light source. In each short-time directional lighting array, the light emitted by each second light source converges in the convergence area, after being reflected by the second lens corresponding to itself. The convergence of the light emitted by each second light source in the convergence area means that the light spots which are formed by lights in the convergence area can be at least partially overlapped, independent of each other without overlapped, or completely overlapped (this one has the best shadowless effect). The convergence area is located in the area of the single patient support. In this embodiment, each position in the convergence area can be illuminated by the light emitted by each second lens. When the medical staff examines the patient 6, not all the second lenses are completely blocked by the head of the medical staff, and the light emitted by some of the second lenses is not blocked. Therefore, the light emitted by these second lenses can also be projected to the convergence area. In this way, there is basically no umbra area in the convergence area on the single patient support 5.

In one embodiment, there are a plurality of second lenses 312, such as eight second lenses 312, in each short-time directional lighting array. The plurality of second lenses 312 in each short-time directional lighting array are arranged along a longitudinal straight line of the mounting framework 35.

In one embodiment, each short-time directional lighting array further includes a flange at both lateral sides of the emergent surface of the second lens. The flange extends along the arrangement direction of the plurality of the second lenses, and each second lens is located between the two flanges. The flange can improve the structural strength of the short-time directional lighting array.

The structure of the second lens in this embodiment can be identical to that of the first lens in the first embodiment.

In one embodiment, the light emitted by the long-time directional lighting array is reflected from the ceiling and then illuminates the area of the single patient support. The long-time directional lighting array includes multiple third light sources, and the third light source includes a third cold white light source and a third warm white light source. That is to say, in this embodiment, the long-time directional lighting array may not be provided with a lens. In this embodiment, there are multiple third light sources. Among them, some of the third light sources are the third cold white light sources, the light of the third cold white light source is cold white light, and the others of the third light sources 311 are the third warm white light sources, and the light of the third warm white light source is warm white light. In this embodiment, the light emitted from the whole long-time directional lighting array is the mixed light of the third cold white light source and the third warm white light source. For example, the color temperature of the third warm white light source is 2200K, and the color temperature of the third cold white light source is 6500K.

The color temperature and illuminance of the overall light of the directional lighting apparatus are the mixed color temperature and mixed illuminance of all the third light sources 311 which are turned on. In one embodiment, the color temperature is adjusted by changing a turn-on ratio of the third light source with different color temperatures. For example, the number of the turn-on third cold white light source is 12, and the number of the turn-on third warm white light source is 12. At this time, the ratio between the turn-on numbers of the third light sources with two color temperatures is 1:1, which corresponds to one color temperature of the long-time directional lighting array. When it is necessary to adjust the color temperature, the turn-on number of the third cold white light source and the turn-on number of the third warm white light source can be adjusted. For example, the turn-on number of the third cold white light sources is 10, and the turn-on number of the third warm white light source is 12. At this time, the ratio between the turn-on numbers of the third light sources with two color temperatures is 5: 6. At this time, the color temperature corresponding to another color temperature of the long-time directional lighting array, that is, the color temperature of the long-time directional lighting array, has changed.

For example, the illuminance of the long-time directional lighting array is adjusted by changing a current corresponding to the third light source. For example, by increasing the current corresponding to the third light source, the illuminance of the third light source increases, and accordingly, the illuminance of the overall long-time directional lighting array also increases.

It is understandable that the illuminance and color temperature can be adjusted separately or simultaneously. For example, the color temperature of the long-time directional lighting array can be adjusted without changing its illuminance, by just changing the ratio between the turn-on numbers of the third light sources with different color temperatures without changing the current of the third light source. Just the illuminance of the long-time directional lighting array can be adjusted by just changing the current of the third light source without changing the ratio between the turn-on numbers of the third light sources with different color temperatures. Both the illuminance and color temperature can be adjusted simultaneously, by changing the ratio between the turn-on numbers of the third light sources with different color temperatures and the current of the third light source, simultaneously.

In an embodiment, at least one short-time directional lighting array includes at least one first short-time directional lighting array and at least one second short-time directional lighting array. A convergence area of the at least one first short-time directional lighting array is a first short-time convergence area, and a convergence area of the at least one second short-time directional lighting array is a second short-time convergence area. The first short-time convergence area and the second short-time convergence area correspond to different body parts of patient lying in the area of the single patient support. That is to say, in this embodiment, there are multiple short-time directional lighting arrays, and some of which is the first short-time directional lighting array, while the others are the second short-time directional lighting array.

The processor is further configured to independently control the first short-time directional lighting array or the second short-time directional lighting array through a short-time directional drive controller according to the instruction from the user. In other words, the lighting of different body parts of the patient can be controlled independently. For example, the first short-time convergence area corresponds to the head area of the patient on the patient support, the second short-time convergence area corresponds to the trunk area of the patient on the patient support, and the lighting of the head area and the trunk area can be controlled separately to reduce the influence on the patient.

In an embodiment, the mounting framework includes a short-time lighting framework plate for mounting the short-time directional lighting array, wherein the short-time lighting framework plate includes a first short-time lighting framework plate and a second short-time lighting framework plate which are connected with each other. The first short-time directional lighting array is mounted at a mounting surface of the first short-time lighting framework plate, and the second short-time directional lighting array is mounted at a mounting surface of the second short-time lighting framework plate. A normal of the mounting surface of the first short-time lighting framework plate intersects a normal of the mounting surface of the second short-time lighting framework plate, and the intersection point is located inside an space between the lighting system and the area of the single patient support. That is to say, at least one of the mounting surfaces of the first short-time lighting framework plate and the second short-time lighting framework plate inclines in the direction of the other. In this way, the first short-time directional lighting array and the second short-time directional lighting array have inclination angles, so that the first short-time directional lighting array and the second short-time directional lighting array have different convergence areas.

In one embodiment, the lighting system further includes a housing, which is arranged around the mounting framework. The housing forms the external surface of the lighting system, which can cover the structure which is arranged inside the mounting framework and improve the appearance of the lighting system. The housing further includes a third light transmission surface arranged below the short-time directional lighting array and a fourth light transmission surface arranged above the long-time directional lighting array. The light emitted by the short-time directional lighting array directly illuminates the area of the single patient support through the third light transmission surface, and the light emitted by the long-time directional lighting array directly illuminates the ceiling of the area of the single patient support and then is reflected back to the area of the single patient support. In other words, the light emitted by the short-time directional lighting array passes through the third light transmission surface and leaves the lighting system, that is, this light does not need to be reflected or transmitted again, and can directly reach the area of the single patient support. The distance between two endpoints of the third light transmission surface is greater than or equal to 40 cm, that is, the linear distance between at least two endpoints of the third light transmission surface is greater than 40 cm. This size is much larger than the head size of ordinary adults. When the medical staff examines the patient 6 on the patient support 5 under the lighting system 3, the third light transmission surface is never completely blocked by the medical staff, and some lights passing through the third light transmission surface can always reach the area of the patient support.

For example, in an embodiment, the third light transmission surface is roughly rectangular, the width of the third light transmission surface along the mounting framework is greater than or equal to 30 cm, and the length of the third light transmission surface along the mounting framework is greater than or equal to 42 cm. That is, the area of the third light transmission surface is greater than or equal to 1260 cm². It can be understood that the shape of the third light transmission surface can also be other shapes, such as, ellipse, pentagon, etc.

In one embodiment, the lighting system further includes a housing, a main control board and a power interface which are connected with each other. The housing is arranged around the mounting framework. The housing forms the external surface of the lighting system, which can cover the structure arranged inside the mounting framework, and improve the appearance of the lighting system. The housing further includes a third light transmission surface arranged below the short-time directional lighting array and a fourth light transmission surface arranged above the long-time directional lighting array. The light emitted by the short-time directional lighting array directly illuminates the area of the single patient support through the third light transmission surface, and the light emitted by the long-time directional lighting array directly illuminates the ceiling of the area of the single patient support and then is reflected back to the area of the single patient support. The main control board is arranged inside the housing and the power interface is arranged at the housing. That is, the light emitted by the short-time directional lighting array passes through the third light transmission surface and leaves the lighting system, that is, this light does not need to be reflected or transmitted again, and can directly reach the area of the single patient support. The lighting system of this embodiment can be used as an independent product, that is, the lighting system 3 is an independent packaging structure. When using, the user just need to plug a power cord of the lighting system 3 into the power interface and power it on.

It should be noted that the parts of the housing 34 that do not require light transmission are made of opaque materials. The materials of the housing 34 at the first light transmission surface 341 include but are not limited to the following materials: polymethylmethacrylate (commonly known as organic glass), polystyrene, polycarbonate, styrene acrylonitrile, etc.

It should be noted that in other embodiments, the lighting system can also be used together with other products, for example, the lighting system can be mounted on the wall, or integrated with a mechanical device. In the embodiment of this disclosure, the lighting system 3 is described as an example integrated with the medical suspension device.

In one embodiment, the configuration information of the default lighting mode includes a plurality of configuration information sets, and each configuration information set includes multiple pieces of default color temperature configuration information and multiple pieces of default illuminance configuration information. Where, during at least one color temperature working hour, the default color temperature configuration parameters corresponding to the color temperature working hour in at least two default color temperature configuration parameter sets are different, or during at least one illuminance working hour associated with the default illuminance configuration information, the default illuminance configuration parameters corresponding to the illuminance working hour in at least two default illuminance configuration parameter sets are different. The processor is configured to determine a configuration information set according to the instruction from the user, dynamically adjust the long-time directional lighting array according to the multiple pieces of default color temperature configuration information and its associated working hour in the determined configuration information set and according to the multiple pieces of default illuminance configuration information and its associated working hour in the determined configuration information set.

Specifically, for example, multiple pieces of default color temperature configuration information and multiple pieces of default illuminance configuration information in each configuration information set constitute a three-dimensional rhythm curve simulating time, illuminance and color temperature, that is, the illuminance and color temperature of the long-time directional lighting array change periodically according to the rhythm curve. For example, the illuminance and color temperature of the long-time directional lighting array change every day according to the above rhythm curve, that is, 24 hours of one day is a rhythm cycle.

When the lighting system works in the default lighting mode, supposing that the processor controls the long-time directional lighting drive controller to execute information in the first configuration information set, that is, the color temperature and illuminance of the long-time directional lighting array change according to the first rhythm curve. After the user inputs a corresponding instruction, such as an instruction to change the illuminance or color temperature, the processor 391 determines another configuration information set corresponding to the instruction, that is, determines another corresponding rhythm curve according to the instruction of the user. Thereafter, the color temperature and illuminance of the long-time directional lighting array change according to the another rhythm curve.

In order to provide the choice of rhythmic illuminance or color temperature for the lighting system of this embodiment, users can adjust according to their actual needs to improve the user experience.

In one embodiment, the configuration information set is a set of color temperature configuration parameters and illuminance configuration parameters corresponding to the simulated circadian rhythm rules. The simulated circadian rhythm rules refer to the simulation of natural light conditions in nature 24 hours a day, which includes color temperature information and illuminance information, and the corresponding relationship among time, illuminance and color temperature, and is formed into a database and stored in memory 392.

In an embodiment, the processor is further configured to regularly invoke the same configuration information set every day and dynamically adjust the long-time directional lighting array every day. That is to say, take 24 hours a day as one rhythm cycle and repeat the default rhythm curve every day. Users can choose to execute other rhythm curves according to their needs.

It can be understood that in other embodiments, the configuration information set in the previous rhythm cycle is automatically executed in the next rhythm cycle. In other words, no matter whether the user selects different configuration information sets in the previous rhythm cycle or not, in the next rhythm cycle, the processor unconditionally controls the long-time lighting drive controller to execute the configuration information set in the previous rhythm cycle.

In one embodiment, the customized lighting mode includes at least one of reading mode, examination mode and light stimulation mode. In the embodiment where the customized lighting mode includes the reading mode, the processor is configured to adjust the illuminance of the short-time directional lighting array in the range of 300lx-2000lx and adjust the color temperature of the short-time directional lighting array in the range of 3000K-5000K and keep the color temperature unchanged, through the short-time directional lighting drive controller, in the reading mode implemented by the short-time directional lighting apparatus. For example, when the patient needs to read on the patient support, high illuminance lighting is not required at this time. The above color temperature range and illuminance range can better meet the lighting needs of patient when reading. In this embodiment, both the illuminance and color temperature cannot be adjusted. In the embodiment where the customized lighting mode includes the examination mode, the processor is configured to adjust the illuminance of the short-time directional lighting array in the 1000lx-10000lx range and adjust the color temperature of the short-time directional lighting array in the range of 3000K-5000K and keep the illuminance color temperature unchanged, through the short-time directional lighting drive controller, in the examination mode implemented by the short-time directional lighting apparatus. For example, when the medical staff needs to examine the patient 6, and the above color temperature and illuminance ranges are required in the trunk area of the patient, and under which color temperature and illuminance ranges the medical staff can perform clinical operations such as intubation. In this embodiment, both the illuminance and color temperature cannot be adjusted. In the embodiment where the customized lighting mode includes the light stimulation mode, the processor is configured to adjust the illuminance of the short-time directional lighting array to gradually change with the highest value of the illuminance in the range of 2000 lx-10000 lx and keep the color temperature unchanged, through the short-time directional lighting drive controller, in the light stimulation mode implemented by the short-time directional lighting apparatus. It can be understood that in the light stimulation mode, the light of the short-time directional lighting array can at least project onto the head area of the patient, so that the eyes of the patient can feel the light.

For example, when it is necessary to wake up the sleeping patient, some of the third light sources provide a time-controllable regional lighting with high illuminance for the head area of the patient. The eyes of the patient can feel the lighting of high illuminance and wake up. For example, in one embodiment, in the light stimulation mode, the illuminance of the head area of patient gradually changes within a preset time period, for example, the sum of the preset light stimulation time periods is 10 minutes to 20 minutes, and the illuminance gradually increases from no light to about 2000 lx to 10000 lx, and then gradually extinguishes.

In this embodiment, the structure of the second lens can be the same as that of the first lens in the first embodiment.

The structure of the first short-time lighting frame plate is the same as that of the first frame plate in the first embodiment. The second short-time lighting skeleton plate has the same structure as the second skeleton plate in the first embodiment.

Another embodiment of this disclosure provides a medical suspension device. Please refer to FIG.1, the medical suspension device includes a beam assembly 1, a suspension column 2, and a lighting system 3 in any of the above third embodiments.

The beam assembly is arranged the area of the single patient support.

The suspension column is suspended below the beam assembly and is arranged at a side of the area of the single patient support. The suspension column is configured to provide an electrical interface or installation device for configuration and use of a medical device (such as, a ventilator, monitor and other medical device) which is used by the patient 6 in the area of the single patient support. Wherein, electrical interfaces, signal line interfaces, power supply interface, gas source interface, etc., as well as, electrical wires, gas pipelines, electrical components, etc. of medical suspension device are arranged inside the suspension column 2. In some embodiments, a first end of the mounting framework 35 protrudes from a lateral side of the beam assembly 1 in the direction of the area of the single patient support, and a second end of the mounting framework 35 is fixedly connected with the beam assembly 1.

The beam assembly 1 has a large mounting space to mount the lighting system 3. In addition, the beam assembly 1 has a large height relative to the ground, and the mounting of the lighting system 3 at the beam assembly 1 never occupies activity space of the medical staff or placement space of other devices. Furthermore, it is possible to enable the light of the lighting system 3 to have a suitable propagation distance.

There is no limit to the number of the suspension columns 2, which can be one, two or more. In the embodiment of this disclosure, two suspension columns 2 are taken as an example to describe. One patient support 5 is placed in front of the beam assembly 1 and between two suspension column 2 which are used by the patient support 5. It can be understood that in other embodiments, the same one medical suspension device can be simultaneously used by the two patient supports 5 which are in the front and back respectively. In this embodiment, two above-mentioned lighting systems 3 can be arranged at the beam assembly 1, and each lighting system 3 is used for one corresponding patient support 5.

In one embodiment, the input/output interface includes a display controller and a display panel which are connected with itself. The display panel is provided with a switch control element and an adjustment control element for the customized lighting mode, and an adjustment control element for the default lighting mode. The adjustment control element for the customized lighting mode is configured to adjust the illuminance, and the adjustment control element for the default lighting mode is configured to adjust the illuminance and/or color temperature. The processor is further configured to determine an instruction from a user through control element information of the display panel which is selected by the user and identified by the display controller. When the long-time directional lighting apparatus works in the default lighting mode, it further responds to the instruction from the user of the switch control element or adjustment control element for the customized lighting mode.

Specifically, when the short-time directional lighting apparatus works in the customized lighting mode, if the user triggers the adjustment control element corresponding to the default lighting mode, the processor further responds to the instruction from the user. Similarly, when the long-time directional lighting apparatus works in the default lighting mode, if the user triggers the switch control element or adjustment control element for the customized lighting mode, the process further responds to the instruction from the user.

In a specific embodiment, the adjustment control element or the switch control element on the display panel can be mechanical keys, as well as, virtual buttons, touch keys arranged on the display screen, etc., which are not limited here.

In one embodiment, the input/output interface is arranged at the suspension column to improve the integration of the medical suspension device, and facilitate the information interaction between the medical staff and the input/output interface, as well as the operation of the medical staff.

It should be noted that, in an embodiment, please refer to FIG.21 again. The display panel 382 is further provided with a switch control element 381c corresponding to the default lighting mode, and the display panel 382 is provided with a switch control element 381a and an adjustment control element 381b corresponding to each customized lighting mode, that is, each customized lighting mode corresponds to one switch control element 381a and one adjustment control element 381b.

In one embodiment, the lighting system further includes a main control board. The long-time directional lighting drive controller 317 and the short-time directional lighting drive controller 318 are arranged at the main control board. The long-time directional lighting array is connected with the long-time directional lighting drive controller 317 through a cable. The short-time directional lighting array is connected with the short-time directional lighting drive controller 318 through a cable. The main control board 33 is arranged inside the beam assembly 1, and one end of the cable passes through the mounting framework 35 and extends into the beam assembly 1. In this embodiment, the main control board 33 is arranged inside the beam assembly 1, and the beam assembly 1 has a large space to facilitate the heat dissipation of the main control board 33.

In another embodiment, the main control board 33 is arranged inside the mounting framework 35. The mounting framework 35 is provided with an accommodation cavity 35a, and the main control board 33 is arranged inside the accommodation cavity 35a. In this way, the main control board 33 can be limited inside the mounting framework 35, so as to prevent the main control board 33 from protruding from the outer surface of the mounting framework 35 and being rubbed by other parts.

In one embodiment, the mounting framework 35 is a metal piece, that is, the material of the mounting framework 35 is metal. By using the thermal conductivity of the metal, the heat generated by the heating elements in the lighting system 3 can be dissipated in time. For example, the heat generated by the main control board 33 and the light source can be dissipated in time. The specific types of metal materials for mounting the framework 35 are not limited, as long as they can conduct heat, for example, copper, iron, aluminum, etc. The metal materials can be pure metal or alloy. The mounting framework 35 can be made of the same metal for the whole structure, or different metals for different parts. There is no restriction here.

Another embodiment of this disclosure provides a lighting control method which is applied to provide a lighting system configured to provide lighting for an area of a single patient support; wherein the lighting system illuminates the area of the single patient support directly or after being reflected by other medium, and includes a processor, a memory and a control panel. It should be noted that the lighting system can be the lighting system in any of the above first or third embodiments.

The lighting control method includes following steps:
S1, powering on the lighting system;
S2, invoking configuration information of a default lighting mode in the memory, wherein the configuration information of the default lighting mode includes multiple pieces of default color temperature configuration information and multiple pieces of default illuminance configuration information; wherein the multiple pieces of default color temperature configuration information are associated with different working hours and the multiple pieces of default illuminance configuration information are associated with different working hours;
S3, adjusting color temperature and illuminance of at least some directional lighting arrays in the lighting system according to the configuration information of the default lighting mode;
S4, receiving an instruction from a user through the control panel;
S5, determining a customized lighting mode according to the instruction from the user;
S6, invoking the configuration information of the customized lighting mode in the memory, wherein the configuration information of the customized lighting mode at least includes customized illuminance configuration information; and
S7, adjusting the illuminance of at least some directional lighting arrays in the lighting system according to the configuration information of the customized lighting mode.

In one embodiment, the directional lighting array which is adjusted according to the configuration information of the default lighting mode is the same as the directional lighting array which is adjusted according to the configuration information of the customized lighting mode; wherein adjusting the illuminance of at least some directional lighting arrays in the lighting system according to the configuration information of the customized lighting mode, includes following steps:
switching from the default lighting mode to the customized lighting mode;
adjusting the illuminance of the directional lighting array according to the configuration information of the customized lighting mode;
adjusting the illuminance and color temperature of the directional lighting array according to the configuration information of the default lighting mode until a preset execution period in the instruction from the user is exceeded or a cancellation instruction is received.

In this embodiment, the same directional lighting array is used to realize the lighting under the customized lighting mode and the default lighting mode, only one of the customized lighting mode and the default lighting mode can be selected. The customized lighting mode and the default lighting mode cannot coexist.

It should be noted that, adjusting the illuminance and color temperature of the directional lighting array according to the configuration information of the default lighting mode until a preset execution period in the instruction from the user is exceeded or a cancellation instruction is received, includes following embodiments.

In the first embodiment, the directional lighting apparatus is controlled to return to the default lighting mode when a cancellation instruction is received from the user. That is, in this embodiment, the memory does not store a preset execution period for the customized lighting mode. The working hours of the customized lighting mode depend on the actual requirement of the user. When the user needs to turn off the current customized lighting mode, the user inputs the cancellation instruction. Then the directional lighting apparatus is controlled to return to the default lighting mode. In the second embodiment, the directional lighting apparatus is controlled to return to the default lighting mode when the execution of the customized lighting mode is complete. In this embodiment, the memory stores the preset execution period for the customized lighting mode. When the working hour of the customized lighting mode reaches the preset execution period, the directional lighting apparatus is automatically controlled to return to the default lighting mode.

In one embodiment, a long-time directional lighting array is adjusted according to the configuration information of the default lighting mode, and a short-time directional lighting array is adjusted according to the configuration information of the customized lighting mode, the long-time directional lighting array and the short-time directional lighting array are different lighting arrays; wherein adjusting the illuminance of at least some directional lighting arrays in the lighting system according to the configuration information of the customized lighting mode, includes following steps:
adjusting the illuminance of the short-time directional lighting array in the lighting system according to the configuration information of the customized lighting mode, until a preset execution period in the instruction from the user is exceeded or a cancellation instruction is received.

In the lighting system of this embodiment, the default lighting mode is realized through the long-time directional lighting array, and the customized lighting mode is realized through the short-time directional lighting array.

In the default lighting mode of the lighting system in the embodiment of this disclosure, the color temperature and illuminance of the long-time directional lighting array can be dynamically and regularly adjusted according to different working hours, that is, the default lighting mode is the rhythmic mode, that is, the default lighting mode is a rhythm mode, that is, the working state of the lighting system is the rhythm state by default, and the lighting system works in the rhythm state for a long time. In this embodiment, the customized lighting mode and the default lighting mode can exist at the same time, that is, at the same time, both the long-time directional lighting array and the short-time directional lighting array can project light into the area of the single patient support.

It should be noted that controlling the short-time directional lighting array to execute the selected customized lighting mode until the cancellation instruction is received or the execution is complete, can include at least one of the two following embodiments.

In the first embodiment, when the cancellation instruction is received from the user, the short-time directional lighting array stops executing the selected customized lighting mode. That is, in this embodiment, the memory does not store a preset execution period for the customized lighting mode. The working hours of the customized lighting mode depend on the actual requirement of the user. When the user needs to turn off the current customized lighting mode, the user inputs the cancellation instruction. Then the short-time directional lighting array stops emitting light.

In the second embodiment, the short-time directional lighting array stops executing the selected customized lighting mode, when the selected customized lighting mode is completed. That is, in this embodiment, the memory stores a preset execution period for the customized lighting mode. When the working hour of the customized lighting mode reaches the preset execution period, the short-time directional lighting array automatically stops emitting light.

In one embodiment, when the short-time directional lighting array works in the customized lighting mode, the color temperature and illuminance of the long-time directional lighting array are further adjusted according to the configuration information of the default lighting mode. In this embodiment, even if the short-time directional lighting array works in the customized lighting mode, the color temperature and illuminance of the long-time directional lighting array can also be adjusted, that is, the long-time directional lighting array can also show rhythmic changes.

In one embodiment, when the short-time directional lighting array works in the customized lighting mode, the long-time directional lighting array is turned off until the preset execution period in the instruction from the user is exceeded or the cancellation instruction is received, then the short-time directional lighting array is turned off and the color temperature and illuminance of the long-time directional lighting array are adjusted again according to the configuration information of the default lighting mode. In this embodiment, the short-time directional lighting array and the long-time directional lighting array work alternatively. When exiting the customized lighting mode of the short-time directional lighting array, the lighting system automatically switches to the default lighting mode, that is, the state in which the long-time directional lighting array works but the short-time directional lighting array does not work.

The embodiments/embodiments provided in this disclosure can be combined with each other without contradiction.

The above are only preferred embodiments of this disclosure and are not intended to limit this disclosure. For those skilled in the art, this disclosure can have various changes and modifications.

## Claims

1. A lighting system (3), comprising:
a directional lighting apparatus, which comprises a directional lighting drive controller (315) and at least one directional lighting array (31a), wherein a light emitted by the at least one directional lighting array (31a) illuminates an area of a single patient support (5), directly or after being reflected by other medium;
a mounting framework (35), which is configured to fix the directional lighting array (31a);
an input/output interface (38), which is configured to receive an instruction from a user;
a memory (392), which is configured to store configuration information of a default lighting mode and at least one customized lighting mode, wherein the configuration information of the default lighting mode comprises multiple pieces of default color temperature configuration information which are associated with different working hours and/or multiple pieces of default illuminance configuration information which are associated with different working hours, wherein the configuration information of the customized lighting mode at least comprises customized illuminance configuration information; and
a processor (391), which is configured to control, when receiving a selection instruction for the at least one customized lighting mode from the user, the directional lighting apparatus to be switched from the default lighting mode, which is in operation, to a selected customized lighting mode, and to control, when a cancellation instruction is received from the user or the selected customized lighting mode is completed, the directional lighting apparatus to return to the default lighting mode, **characterized in that**, the at least one directional lighting array (31a) comprises at least one first directional lighting array (31a') and at least one second directional lighting array (31a"), the processor (391) is further configured to control the first directional lighting array (31a') and the second directional lighting array (31a") through the directional lighting drive controller (315) independently, according to the instruction from the user; wherein a convergence area of the at least one first directional lighting array (31a') is a first convergence area (30a), a convergence area of the at least one second directional lighting array (31a") is a second convergence area (30b); wherein the first convergence area (30a) and the second convergence area (30b) respectively correspond to different parts of the single patient support (5).

2. The lighting system (3) according to claim 1 wherein the processor (391) is further configured to, in the default lighting mode, invoke the default color temperature configuration information and the default illuminance configuration information corresponding to different working hours, and adjust color temperature and illuminance of the directional lighting array (31a) through the directional lighting drive controller (315), according to the invoked default color temperature configuration information and the invoked default illuminance configuration information; and the processor (391) is further configured to, in the customized lighting mode, invoke the customized illuminance configuration information corresponding to different working hours, and adjust the illuminance of the directional lighting array (31a) through the directional lighting drive controller (315), according to the customized illuminance configuration information; or
the configuration information of the default lighting mode comprises a plurality of configuration information sets, and each configuration information set comprises the multiple pieces of default color temperature configuration information and the multiple pieces of default illuminance configuration information; wherein, during at least one color temperature working hour which is associated with the default color temperature configuration information, default color temperature configuration parameters, which correspond to the color temperature working hour, in at least two default color temperature configuration parameter sets, are different; or during at least one illuminance working hour which is associated with the default illuminance configuration information, default illuminance configuration parameters, which correspond to the illuminance working hour, in at least two default illuminance configuration parameter sets, are different; the processor (391) is further configured to determine the configuration information set according to the instruction from the user, and dynamically adjust the directional lighting array (31a), according to the multiple pieces of default color temperature configuration information in the determined configuration information set and the working hour which is associated with the multiple pieces of default color temperature configuration information, and according to the multiple pieces of default illuminance configuration information in the determined configuration information set and the working hour which is associated with the multiple pieces of default illuminance configuration information.

3. The lighting system (3) according to claim 1, wherein the directional lighting array (31a) comprises a plurality of first light sources (311) and a plurality of first lenses (312), each first lens (312) corresponds to at least one of the first light sources (311), wherein in each directional lighting array (31a), a light emitted by each first light source (311) converges in a convergence area after being reflected by the first lens (312) which is correspondingly arranged; wherein the convergence area is located at the area of the single patient support (5); preferably, each first light source (311), which corresponds to the first lens (312), comprises a first cold white light source and a first warm white light source; or in each directional lighting array (31a), some of the first light sources (311), which correspond to the first lens (312), are first cold white light source(s), other first light sources (311), which correspond to the first lens (312), are first warm white light source(s); or
the at least one directional lighting array (31a) comprises a direct directional lighting array and a reflective directional lighting array (321), wherein a light emitted by the direct directional lighting array directly illuminates the area of the single patient support (5), and a light emitted by the reflective directional lighting array (321) illuminates the area of the single patient support (5) after being reflected through a ceiling of the area of the single patient support (5).

4. The lighting system (3) according to claim 1, wherein the mounting framework (35) comprises a first framework plate (351) and a second framework plate (352) which are connected with each other; wherein the first directional lighting array (31a') is mounted at a first mounting surface (351a) of the first framework plate (351), the second directional lighting array (31a") is mounted at a second mounting surface (352a) of the second framework plate (352); wherein a normal of the first mounting surface (351a) intersects with a normal of the second mounting surface (352a), and an intersection point is located inside a space between the lighting system (3) and the area of the single patient support (5).

5. The lighting system (3) according to claim 1, wherein the customized lighting mode comprises at least one of reading mode, examination mode and light stimulation mode;
when the customized lighting mode comprises the reading mode, the processor (391) is further configured to, in the reading mode, adjust illuminance of the directional lighting array (31a) in a range of 3001x-20001x, adjust color temperature of the directional lighting array (31a) in a range of 3000K-5000K and keep the color temperature unchanged, through the directional lighting drive controller (315); or
when the customized lighting mode comprises the examination mode, the processor (391) is further configured to, in the examination mode, adjust illuminance of the directional lighting array (31a) in a range of 1000lx-l0000lx, adjust color temperature of the directional lighting array (31a) in a range of 3000K-5000K and keep the color temperature unchanged, through the directional lighting drive controller (315); or
when the customized lighting mode comprises the light stimulation mode, the processor (391) is further configured to, in the light stimulation mode, adjust illuminance of the directional lighting array (31a) to gradually change with a highest value of the illuminance in a range of 2000lx-10000lx and keep color temperature of the directional lighting array (31a) unchanged, through the directional lighting drive controller (315).

6. The lighting system (3) according to claim 1, wherein the lighting system (3) further comprises a housing which is arranged around the mounting framework (35), wherein the housing comprises a first light transmission surface which is arranged at a bottom side of the directional lighting array (31a), the light emitted by the directional lighting array (31a) directly illuminates the area of the single patient support (5) through the first light transmission surface, wherein a distance between two endpoints of the first light transmission surface is greater than or equal to 40cm; or
the lighting system (3) further comprises a housing which is arranged around the mounting framework (35), and a main control board and a power interface which are connected with each other; wherein the housing comprises a first light transmission surface which is arranged at a bottom side of the directional lighting array (31a), the light emitted by the directional lighting array (31a) directly illuminates the area of the single patient support (5) through the first light transmission surface, the main control board is arranged inside the housing, and the power interface is arranged at the housing.

7. A lighting control method which is applied to the lighting system (3) according to any one of claims 1 to 6; wherein the lighting control method comprises:
powering on the lighting system (3);
invoking configuration information of a default lighting mode in the memory (392), wherein the configuration information of the default lighting mode comprises multiple pieces of default color temperature configuration information and multiple pieces of default illuminance configuration information; wherein the multiple pieces of default color temperature configuration information are associated with different working hours and the multiple pieces of default illuminance configuration information are associated with different working hours;
adjusting color temperature and illuminance of at least part of a directional lighting array (31a) in the lighting system (3) according to the configuration information of the default lighting mode;
receiving an instruction from a user through the control panel;
determining a customized lighting mode according to the instruction from the user;
invoking configuration information of the customized lighting mode in the memory (392), wherein the configuration information of the customized lighting mode at least comprises customized illuminance configuration information; and
adjusting illuminance of at least part of the directional lighting array (31a) in the lighting system (3) according to the configuration information of the customized lighting mode.

8. The lighting control method according to claim 7, wherein
the directional lighting array (31a), which is adjusted according to the configuration information of the default lighting mode, is same as the directional lighting array (31a), which is adjusted according to the configuration information of the customized lighting mode; wherein adjusting illuminance of at least part of the directional lighting array (31a) in the lighting system (3) according to the configuration information of the customized lighting mode, comprises: switching from the default lighting mode to the customized lighting mode; adjusting the illuminance of the directional lighting array (31a) according to the configuration information of the customized lighting mode; and adjusting the illuminance and the color temperature of the directional lighting array (31a) according to the configuration information of the default lighting mode, until a preset execution period in the instruction from the user is exceeded or a cancellation instruction is received; or
the directional lighting array (31a), which is adjusted according to the configuration information of the default lighting mode, is a long-time directional lighting array; and the directional lighting array (31a), which is adjusted according to the configuration information of the customized lighting mode, is a short-time directional lighting array; wherein the long-time directional lighting array and the short-time directional lighting array are different; wherein adjusting illuminance of at least part of the directional lighting array in the lighting system (3) according to the configuration information of the customized lighting mode, comprises: adjusting illuminance of the short-time directional lighting array in the lighting system (3) according to the configuration information of the customized lighting mode, until a preset execution period in the instruction from the user is exceeded or a cancellation instruction is received.

## Patentansprüche

1. Ein Beleuchtungssystem (3) mit
einem Gerät zur gerichteten Beleuchtung, das ein Steuergerät (315) für die gerichtete Beleuchtung und mindestens eine gerichtete Beleuchtungseinheit (31a) umfasst, von der ein ausgestrahltes Licht den Betreuungsbereich eines einzelnen Patienten (5) entweder direkt oder über ein anderes Medium reflektiert beleuchtet;
einem Montagerahmen (35) zur Befestigung der gerichteten Beleuchtungseinheit (31a);
einer Ein-/Ausgabeschnittstelle (38), die für den Empfang einer Anweisung von einem Benutzer konzipiert ist;
einem Speicher (392) für die Speicherung von Konfigurationsinformationen eines Standardbeleuchtungsmodus und mindestens eines benutzerdefinierten Beleuchtungsmodus, wobei die Konfigurationsinformationen des Standardbeleuchtungsmodus mehrere Elemente der Standardfarbtemperatur-Konfigurationsinformationen umfassen, die mit verschiedenen Arbeitszeiten verknüpft sind, und/oder mehrere Elemente der Standardbeleuchtungsstärke-Konfigurationsinformationen, die mit verschiedenen Arbeitszeiten verknüpft sind, wobei die Konfigurationsinformationen des benutzerdefinierten Beleuchtungsmodus zumindest benutzerdefinierte Beleuchtungsstärke-Konfigurationsinformationen umfassen; und
einem Prozessor (391), der dazu konfiguriert ist, beim Empfang einer Auswahlanweisung für zumindest einen benutzerdefinierten Beleuchtungsmodus durch den Benutzer die gerichtete Beleuchtungseinheit von dem aktuell betriebenen Standardbeleuchtungsmodus auf den ausgewählten benutzerdefinierten Beleuchtungsmodus umzuschalten und beim Empfang einer Stornierungsanweisung durch den Benutzer oder bei Abschluss des ausgewählten benutzerdefinierten Beleuchtungsmodus die gerichtete Beleuchtungseinheit in den Standardbeleuchtungsmodus zurückzusetzen, mit der Eigenschaft, dass die mindestens eine gerichtete Beleuchtungseinheit (31a) zumindest eine erste gerichtete Beleuchtungseinheit (31a) und eine zweite gerichtete Beleuchtungseinheit (31a") beinhaltet, wobei der Prozessor (391) ferner dazu konfiguriert ist, die erste gerichtete Beleuchtungseinheit (31a') und die zweite gerichtete Beleuchtungseinheit (31a") über das Steuergerät (315) für die gerichtete Beleuchtung unabhängig voneinander entsprechend der Anweisung des Benutzers zu steuern; wobei ein Konvergenzbereich zumindest einer ersten gerichteten Beleuchtungseinheit (31a') einem ersten Konvergenzbereich (30a) entspricht, ein Konvergenzbereich zumindest einer zweiten gerichteten Beleuchtungseinheit (31a") einem zweiten Konvergenzbereich (30b) entspricht; wobei der erste Konvergenzbereich (30a) und der zweite Konvergenzbereich (30b) jeweils unterschiedlichen Betreuungsbereichen eines einzelnen Patienten (5) entsprechen.

2. Beleuchtungssystem (3) nach Anspruch 1, wobei der Prozessor (391) ferner dazu konfiguriert ist, im Standardbeleuchtungsmodus die Konfigurationsinformationen bezüglich der Standardfarbtemperatur und der Standardbeleuchtungsstärke abzurufen, die jeweils verschiedenen Arbeitszeiten entsprechen, und die Farbtemperatur sowie die Beleuchtungsstärke der gerichteten Beleuchtungseinheit (31a) über das Steuergerät (315) entsprechend den abgerufenen Standardfarbtemperatur- und Standardbeleuchtungsstärke-Konfigurationsinformationen anzupassen; im benutzerdefinierten Beleuchtungsmodus die benutzerdefinierten Beleuchtungsstärke-Konfigurationsinformationen abzurufen, die verschiedenen Arbeitszeiten entsprechen, und die Beleuchtungsstärke der gerichteten Beleuchtungseinheit (31a) über das Steuergerät (315) entsprechend den benutzerdefinierten Beleuchtungsstärke-Konfigurationsinformationen anzupassen; oder
die Konfigurationsinformationen des Standardbeleuchtungsmodus umfassen mehrere Konfigurationsinformationssätze, wobei jeder Konfigurationsinformationssatz mehrere Elemente der Konfigurationsinformationen bezüglich Standardfarbtemperatur und Standardbeleuchtungsstärke umfasst; wobei während mindestens einer Farbtemperatur-Arbeitszeit, die mit den Konfigurationsinformationen zur Standardfarbtemperatur verknüpft ist, die Standardfarbtemperatur-Konfigurationsparameter, die der Farbtemperatur-Arbeitszeit in mindestens zwei Standardfarbtemperatur-Konfigurationsparametersätzen entsprechen, unterschiedlich sind; oder während mindestens einer Beleuchtungsstärke-Arbeitszeit, die mit den Konfigurationsinformationen zur Standardbeleuchtungsstärke verknüpft ist, die Standardbeleuchtungsstärke-Konfigurationsparameter, die der Beleuchtungsstärke-Arbeitszeit in mindestens zwei Standardbeleuchtungsstärke-Konfigurationsparametersätzen entsprechen, unterschiedlich sind;
der Prozessor (391) weiterhin dazu konfiguriert ist, den Konfigurationsinformationssatz gemäß der Anweisung des Benutzers zu bestimmen und die gerichtete Beleuchtungseinheit (31a) dynamisch entsprechend den mehreren Elementen der Standardfarbtemperatur-Konfigurationsinformationen in dem bestimmten Konfigurationsinformationssatz sowie der Arbeitszeit, die mit den mehreren Elementen der Standardfarbtemperatur-Konfigurationsinformationen verknüpft ist, und entsprechend den mehreren Elementen der Standardbeleuchtungsstärke-Konfigurationsinformationen in dem bestimmten Konfigurationsinformationssatz sowie der Arbeitszeit, die mit den mehreren Elementen der Standardbeleuchtungsstärke-Konfigurationsinformationen verknüpft ist, anzupassen.

3. Beleuchtungssystem (3) nach Anspruch 1, wobei die gerichtete Beleuchtungseinheit (31a) eine Vielzahl von ersten Lichtquellen (311) und eine Vielzahl von ersten Linsen (312) umfasst, jede erste Linse (312) zumindest einer der ersten Lichtquellen (311) entspricht, wobei in jeder gerichteten Beleuchtungseinheit (31a) das von jeder ersten Lichtquelle (311) emittierte Licht nach Reflexion durch die zugehörige erste Linse (312) in einem Konvergenzbereich gebündelt wird; wobei der Konvergenzbereich sich im Betreuungsbereich eines einzelnen Patienten (5) befindet; vorzugsweise umfasst jede erste Lichtquelle (311), die der ersten Linse (312) entspricht, eine erste kaltweiße Lichtquelle und eine erste warmweiße Lichtquelle; oder in jeder gerichteten Beleuchtungseinheit (31a) sind einige der ersten Lichtquellen (311), die der ersten Linse (312) entsprechen, erste kaltweiße Lichtquellen, andere erste Lichtquellen (311), die der ersten Linse (312) entsprechen, sind erste warmweiße Lichtquellen; oder
die mindestens eine gerichtete Beleuchtungseinheit (31a) umfasst eine direkte gerichtete Beleuchtungseinheit und eine reflektierende gerichtete Beleuchtungseinheit(321), wobei das von der direkten gerichteten Beleuchtungseinheit emittierte Licht direkt den Betreuungsbereich eines einzelnen Patienten (5) beleuchtet und das von der reflektierenden gerichteten Beleuchtungseinheit (321) emittierte Licht nach Reflexion über die Decke über dem Bereich der einzelnen Patientenliege (5) den Bereich der einzelnen Patientenliege (5) beleuchtet.

4. Beleuchtungssystem (3) nach Anspruch 1, wobei der Montagerahmen (35) eine erste Rahmenplatte (351) und eine zweite Rahmenplatte (352) aufweist, die miteinander verbunden sind; wobei die erste gerichtete Beleuchtungseinheit (31a') an einer ersten Montagefläche (351a) der ersten Rahmenplatte (351) montiert ist, die zweite gerichtete Beleuchtungseinheit (31a") an einer zweiten Montagefläche (352a) der zweiten Rahmenplatte (352) montiert ist; wobei eine Gerade der ersten Montagefläche (351a) eine Gerade der zweiten Montagefläche (352a) schneidet, und der Schnittpunkt innerhalb eines Raums zwischen dem Beleuchtungssystem (3) und dem Betreuungsbereich eines einzelnen Patienten (5) liegt.

5. Beleuchtungssystem (3) nach Anspruch 1, wobei der benutzerdefinierte Beleuchtungsmodus entweder einen Lesemodus, einen Prüfmodus oder einen Lichtstimulationsmodus beinhaltet;
wenn der benutzerdefinierte Beleuchtungsmodus den Lesemodus beinhaltet, ist der Prozessor (391) weiterhin dazu konfiguriert, über das Steuergerät (315) die Beleuchtungsstärke der gerichteten Beleuchtungseinheit (31a) im Bereich von 300 lx-2000 lx anzupassen, die Farbtemperatur der gerichteten Beleuchtungseinheit (31a) im Bereich von 3000K-5000K anzupassen und die Farbtemperatur unverändert zu halten;
oder
wenn der benutzerdefinierte Beleuchtungsmodus den Prüfmodus beinhaltet, ist der Prozessor (391) weiterhin dazu konfiguriert, über das Steuergerät (315) die Beleuchtungsstärke der gerichteten Beleuchtungsanordnung (31a) im Bereich von 1000 lx-10000 lx anzupassen, die Farbtemperatur der gerichteten Beleuchtungsanordnung (31a) im Bereich von 3000K-5000K anzupassen und die Farbtemperatur unverändert zu halten.
oder
ist der Prozessor (391) weiterhin dazu konfiguriert, über das Steuergerät (315) die Beleuchtungsstärke der gerichteten Beleuchtungsanordnung (31a) so anzupassen, dass sie sich allmählich mit einem höchsten Wert der Beleuchtungsstärke im Bereich von 2000 lx-10000 lx ändert und die Farbtemperatur der gerichteten Beleuchtungsanordnung (31a) unverändert bleibt.

6. Beleuchtungssystem (3) nach Anspruch 1, wobei das Beleuchtungssystem (3) ferner ein Gehäuse für den Montagerahmen (35) mit einer ersten Lichtübertragungsfläche umfasst, die sich an der Unterseite der gerichteten Beleuchtungseinheit (31a) befindet, und das von der gerichteten Beleuchtungseinheit (31a) emittierte Licht direkt durch die erste Lichtübertragungsfläche den Betreuungsbereich eines einzelnen Patienten (5) beleuchtet, wobei der Abstand zwischen zwei Endpunkten der ersten Lichtübertragungsfläche größer oder gleich 40 cm ist;
oder das Beleuchtungssystem (3) ferner ein Gehäuse für den Montagerahmen (35) sowie eine Hauptsteuerplatine und eine Stromschnittstelle umfasst, die miteinander verbunden sind; wobei das Gehäuse eine erste Lichtübertragungsfläche aufweist, die sich an der Unterseite der gerichteten Beleuchtungseinheit (31a) befindet, das von der gerichteten Beleuchtungseinheit (31a) emittierte Licht direkt durch die erste Lichtübertragungsfläche den Betreuungsbereich eines einzelnen Patienten (5) beleuchtet, die Hauptsteuerplatine sich innerhalb des Gehäuses befindet und die Stromschnittstelle am Gehäuse angeordnet ist.

7. Ein Verfahren zur Lichtsteuerung, das auf das Beleuchtungssystem (3) nach einem der Ansprüche 1 bis 6 angewendet wird, wobei das Verfahren Folgendes umfasst:
Einschalten des Beleuchtungssystems (3);
Abrufen der Konfigurationsinformationen eines Standardbeleuchtungsmodus im Speicher (392), wobei die Konfigurationsinformationen des Standardbeleuchtungsmodus mehrere Elemente der Standardfarbtemperatur-Konfigurationsinformationen und mehrere Elemente der Standardbeleuchtungsstärke-Konfigurationsinformationen umfassen; wobei die mehreren Elemente der Standardfarbtemperatur-Konfigurationsinformationen mit verschiedenen Arbeitszeiten verknüpft sind und die mehreren Elemente der Standardbeleuchtungsstärke-Konfigurationsinformationen mit verschiedenen Arbeitszeiten verknüpft sind;
Anpassen der Farbtemperatur und der Beleuchtungsstärke von mindestens einem Element einer gerichteten Beleuchtungseinheit (31a) im Beleuchtungssystem (3) gemäß den Konfigurationsinformationen des Standardbeleuchtungsmodus;
Empfangen einer Anweisung von einem Benutzer über das Bedienfeld;
Bestimmen eines benutzerdefinierten Beleuchtungsmodus gemäß der Anweisung des Benutzers;
Abrufen der Konfigurationsinformationen des benutzerdefinierten Beleuchtungsmodus im Speicher (392), wobei die Konfigurationsinformationen des benutzerdefinierten Beleuchtungsmodus mindestens benutzerdefinierte Beleuchtungsstärke-Konfigurationsinformationen umfassen;
Beleuchtungsstärke von mindestens einem Element der gerichteten Beleuchtungseinheit (31a) im Beleuchtungssystem (3) gemäß den Konfigurationsinformationen des benutzerdefinierten Beleuchtungsmodus.

8. Verfahren zur Lichtsteuerung nach Anspruch 7,
wobei die gerichtete Beleuchtungseinheit (31a), die gemäß den Konfigurationsinformationen des Standardbeleuchtungsmodus angepasst wird, dieselbe ist wie die gerichtete Beleuchtungseinheit (31a), die gemäß den Konfigurationsinformationen des benutzerdefinierten Beleuchtungsmodus angepasst wird; wobei das Anpassen der Beleuchtungsstärke von mindestens einem Element der gerichteten Beleuchtungseinheit (31a) im Beleuchtungssystem (3) gemäß den Konfigurationsinformationen des benutzerdefinierten Beleuchtungsmodus Folgendes umfasst: Umschalten vom Standardbeleuchtungsmodus auf den benutzerdefinierten Beleuchtungsmodus; Anpassen der Beleuchtungsstärke der gerichteten Beleuchtungseinheit (31a) gemäß den Konfigurationsinformationen des benutzerdefinierten Beleuchtungsmodus; und Anpassen der Beleuchtungsstärke und der Farbtemperatur der gerichteten Beleuchtungseinheit (31a) gemäß den Konfigurationsinformationen des Standardbeleuchtungsmodus, bis eine voreingestellte Ausführungsdauer in der Anweisung des Benutzers überschritten wird oder eine Abbruchanweisung empfangen wird; oder
die gerichtete Beleuchtungseinheit (31a), die gemäß den Konfigurationsinformationen des Standardbeleuchtungsmodus angepasst wird, ist eine Langzeit-Beleuchtungseinheit; und die gerichtete Beleuchtungseinheit (31a), die gemäß den Konfigurationsinformationen des benutzerdefinierten Beleuchtungsmodus angepasst wird, ist eine Kurzzeit-Beleuchtungseinheit; wobei die Langzeit-Beleuchtungseinheit und die Kurzzeit-Beleuchtungseinheit unterschiedlich sind; wobei das Anpassen der Beleuchtungsstärke von mindestens einem Element der gerichteten Beleuchtungseinheit im Beleuchtungssystem (3) gemäß den Konfigurationsinformationen des benutzerdefinierten Beleuchtungsmodus Folgendes umfasst: Anpassen der Beleuchtungsstärke der Kurzzeit-Beleuchtungseinheit im Beleuchtungssystem (3) gemäß den Konfigurationsinformationen des benutzerdefinierten Beleuchtungsmodus, bis eine voreingestellte Ausführungsdauer in der Anweisung des Benutzers überschritten wird oder eine Abbruchanweisung empfangen wird.

## Revendications

1. Système d'éclairage (3), comprenant :
un appareil d'éclairage directionnel, lequel comprend une unité de commande d'excitation d'éclairage directionnel (315) et au moins un réseau d'éclairage directionnel (31a), où une lumière émise par le au moins un réseau d'éclairage directionnel (31a) illumine une zone d'un unique support de patient (5), directement ou après avoir été réfléchie par un autre milieu ;
un cadre de montage (35), lequel est configuré pour fixer le réseau d'éclairage directionnel (31a) ;
une interface d'entrée/sortie (38), laquelle est configurée pour recevoir une instruction émanant d'un utilisateur ;
une mémoire (392), laquelle est configurée pour stocker des informations de configuration d'un mode d'éclairage par défaut et d'au moins un mode d'éclairage personnalisé, où les informations de configuration du mode d'éclairage par défaut comprennent de multiples éléments d'information de configuration de température de couleur par défaut lesquels sont associés à différentes heures de travail et/ou de multiples éléments d'information de configuration d'éclairement par défaut lesquels sont associés à différentes heures de travail, où les informations de configuration du mode d'éclairage personnalisé comprennent au moins des informations de configuration d'éclairement personnalisé ; et
un processeur (391), lequel est configuré pour commander, à la réception d'une instruction de sélection pour le au moins un mode d'éclairage personnalisé de l'utilisateur, l'appareil d'éclairage directionnel pour le passer du mode d'éclairage par défaut, lequel est en cours d'utilisation, à un mode d'éclairage personnalisé sélectionné, et pour commander, lorsqu'une instruction d'annulation est reçue de l'utilisateur ou que le mode d'éclairage personnalisé sélectionné est terminé, l'appareil d'éclairage directionnel pour le ramener au mode d'éclairage par défaut, **caractérisé en ce que**, le au moins un réseau d'éclairage directionnel (31a) comprend au moins un premier réseau d'éclairage directionnel (31a') et au moins un second réseau d'éclairage directionnel (31a"), le processeur (391) est en outre configuré pour commander le premier réseau d'éclairage directionnel (31a') et le second réseau d'éclairage directionnel (31a'') par le biais de l'unité de commande d'excitation d'éclairage directionnel (315) indépendamment, selon l'instruction de l'utilisateur; où une zone de convergence du au moins un premier réseau d'éclairage directionnel (31a') est une première zone de convergence (30a), une zone de convergence du au moins un second réseau d'éclairage directionnel (31a") est une seconde zone de convergence (30b) ; où la première zone de convergence (30a) et la seconde zone de convergence (30b) correspondent respectivement à différentes parties de l'unique support de patient (5).

2. Système d'éclairage (3) selon la revendication 1, dans lequel le processeur (391) est en outre configuré pour, dans le mode d'éclairage par défaut, invoquer les informations de configuration de température de couleur par défaut et les informations de configuration d'éclairement par défaut correspondant à différentes heures de travail, et régler la température de couleur et l'éclairement du réseau d'éclairage directionnel (31a) par le biais de l'unité de commande d'excitation d'éclairage directionnel (315), selon les informations de configuration de température de couleur par défaut invoquées et les informations de configuration d'éclairement par défaut invoquées ; et le processeur (391) est en outre configuré pour, dans le mode d'éclairage personnalisé, invoquer les informations de configuration d'éclairement personnalisé correspondant à différentes heures de travail, et régler l'éclairement du réseau d'éclairage directionnel (31a) par le biais de l'unité de commande d'excitation d'éclairage directionnel (315), selon les informations de configuration d'éclairement personnalisé ; ou
les informations de configuration du mode d'éclairage par défaut comprennent une pluralité d'ensembles d'informations de configuration, et chaque ensemble d'informations de configuration comprend de multiples éléments d'information de configuration de température de couleur par défaut et de multiples éléments d'informations de configuration d'éclairement par défaut ; où, durant au moins une heure de travail de température de couleur laquelle est associée aux informations de configuration de température de couleur par défaut, des paramètres de configuration de température de couleur par défaut, lesquels correspondent à l'heure de travail de température de couleur, dans au moins deux ensembles de paramètres de configuration de température de couleur par défaut, sont différents ; ou durant au moins une heure de travail d'éclairement laquelle est associée aux informations de configuration d'éclairement par défaut, des paramètres de configuration d'éclairement par défaut, lesquels correspondent à l'heure de travail d'éclairement, dans au moins deux ensembles de paramètres de configuration d'éclairement par défaut, sont différents ; le processeur (391) est en outre configuré pour déterminer l'ensemble d'informations de configuration selon l'instruction de l'utilisateur, et régler dynamiquement le réseau d'éclairage directionnel (31a), selon les multiples éléments d'information de configuration de température de couleur par défaut dans l'ensemble d'informations de configuration déterminé et l'heure de travail qui est associée aux multiples éléments d'information de configuration de température de couleur par défaut, et selon les multiples éléments d'information de configuration d'éclairement par défaut dans l'ensemble d'informations de configuration déterminé et l'heure de travail qui est associée aux multiples éléments d'information de configuration d'éclairement par défaut.

3. Système d'éclairage (3) selon la revendication 1, dans lequel le réseau d'éclairage directionnel (31a) comprend une pluralité de premières sources de lumière (311) et une pluralité de premières lentilles (312), chaque première lentille (312) correspond à au moins une des premières sources de lumière (311), où dans chaque réseau d'éclairage directionnel (31a), une lumière émise par chaque première source de lumière (311) converge dans une zone de convergence après avoir été réfléchie par la première lentille (312) qui est agencée de manière correspondante ; où la zone de convergence est située au niveau de la zone de l'unique support de patient (5) ; de préférence, chaque première source de lumière (311), qui correspond à la première lentille (312), comprend une première source de lumière blanc froid et une première source de lumière blanc chaud ; ou dans chaque réseau d'éclairage directionnel (31a), certaines des premières sources de lumière (311) qui correspondent à la première lentille (312), sont une (des) première(s) source(s) de lumière blanc froid, d'autres premières sources de lumière (311) qui correspondent à la première lentille (312), sont une (des) première(s) source(s) de lumière blanc chaud ; ou
le au moins un réseau d'éclairage directionnel (31a) comprend un réseau d'éclairage directionnel direct et un réseau d'éclairage directionnel à réflexion (321), où une lumière émise par le réseau d'éclairage directionnel direct illumine directement la zone de l'unique support de patient (5), et une lumière émise par le réseau d'éclairage directionnel à réflexion (321) illumine la zone de l'unique support de patient (5) après avoir été réfléchie par un plafond de la zone de l'unique support de patient (5).

4. Système d'éclairage (3) selon la revendication 1, dans lequel le cadre de montage (35) comprend une première plaque de cadre (351) et une seconde plaque de cadre (352) qui sont reliées l'une à l'autre ; où le premier réseau d'éclairage directionnel (31a') est monté au niveau d'une première surface de montage (351a) de la première plaque de cadre (351), le second réseau d'éclairage directionnel (31a") est monté au niveau d'une seconde surface de montage (352a) de la seconde plaque de cadre (352) ; où une normale de la première surface de montage (351a) coupe une normale de la seconde surface de montage (352a), et un point d'intersection est situé à l'intérieur d'un espace entre le système d'éclairage (3) et la zone de l'unique support de patient (5).

5. Système d'éclairage (3) selon la revendication 1, dans lequel le mode d'éclairage personnalisé comprend au moins un mode parmi un mode de lecture, un mode d'examen et un mode de stimulation lumineuse ;
lorsque le mode d'éclairage personnalisé comprend le mode de lecture, le processeur (391) est en outre configuré pour, dans le mode de lecture, régler l'éclairement du réseau d'éclairage directionnel (31a) dans une plage de 300 lx à 2000 lx, régler une température de couleur du réseau d'éclairage directionnel (31a) dans une plage de 3000 K à 5000 K et garder la température de couleur inchangée, par le biais de l'unité de commande d'excitation d'éclairage directionnel (315) ; ou
lorsque le mode d'éclairage personnalisé comprend le mode d'examen, le processeur (391) est en outre configuré pour, dans le mode d'examen, régler l'éclairement du réseau d'éclairage directionnel (31a) dans une plage de 1000 lx à 10000 lx, régler une température de couleur du réseau d'éclairage directionnel (31a) dans une plage de 3000 K à 5000 K et garder la température de couleur inchangée, par le biais de l'unité de commande d'excitation d'éclairage directionnel (315) ; ou
lorsque le mode d'éclairage personnalisé comprend le mode de stimulation lumineuse, le processeur (391) est en outre configuré pour, dans le mode de stimulation lumineuse, régler l'éclairement du réseau d'éclairage directionnel (31a) de sorte à le faire progressivement varier avec une plus forte valeur de l'éclairement dans une plage de 2000 lx à 10000 lx, et garder la température de couleur du réseau d'éclairage directionnel (31a) inchangée, par le biais de l'unité de commande d'excitation d'éclairage directionnel (315).

6. Système d'éclairage (3) selon la revendication 1, où le système d'éclairage (3) comprend en outre un logement lequel est agencé autour du cadre de montage (35), où le logement comprend une première surface de transmission de lumière laquelle est agencée au niveau d'un côté inférieur du réseau d'éclairage directionnel (31a), la lumière émise par le réseau d'éclairage directionnel (31a) illumine directement la zone de l'unique support de patient (5) par le biais de la première surface de transmission de lumière, où une distance entre deux points d'extrémité de la première surface de transmission de lumière est supérieure ou égale à 40 cm ; ou
le système d'éclairage (3) comprend en outre un logement lequel est agencé autour du cadre de montage (35), et une carte de commande principale et une interface de puissance qui sont reliées l'une à l'autre ; où le logement comprend une première surface de transmission de lumière laquelle est agencée au niveau d'un côté inférieur du réseau d'éclairage directionnel (31a), la lumière émise par le réseau d'éclairage directionnel (31a) illumine directement la zone de l'unique support de patient (5) par le biais de la première surface de transmission de lumière, la carte de commande principale est agencée à l'intérieur du logement, et l'interface de puissance est agencée au niveau du logement.

7. Procédé de commande d'éclairage lequel est appliqué au système d'éclairage (3) selon l'une quelconque des revendications 1 à 6 ; où le procédé de commande d'éclairage comprend les étapes consistant à :
allumer le système d'éclairage (3) ;
invoquer des informations de configuration d'un mode d'éclairage par défaut dans la mémoire (392), où les informations de configuration du mode d'éclairage par défaut comprennent de multiples éléments d'information de configuration de température de couleur par défaut et de multiples éléments d'information de configuration d'éclairement par défaut ; où les multiples éléments d'information de configuration de température de couleur par défaut sont associés à différentes heures de travail et les multiples éléments d'information de configuration d'éclairement par défaut sont associés à différentes heures de travail ;
régler la température de couleur et l'éclairement d'au moins une partie d'un réseau d'éclairage directionnel (31a) dans le système d'éclairage (3) selon les informations de configuration du mode d'éclairage par défaut ;
recevoir une instruction d'un utilisateur par le bais d'un panneau de commande ;
déterminer un mode d'éclairage personnalisé selon l'instruction de l'utilisateur ;
invoquer des informations de configuration du mode d'éclairage personnalisé dans la mémoire (392), où les informations de configuration du mode d'éclairage personnalisé comprennent au moins des informations de configuration d'éclairement personnalisé ; et
régler l'éclairement d'au moins une partie du réseau d'éclairage directionnel (31a) dans le système d'éclairage (3) selon les informations de configuration du mode d'éclairage personnalisé.

8. Procédé de commande d'éclairage selon la revendication 7, dans lequel
le réseau d'éclairage directionnel (31a), qui est réglé selon les informations de configuration du mode d'éclairage par défaut, est le même que le réseau d'éclairage directionnel (31a), qui est réglé selon les informations de configuration du mode d'éclairage personnalisé ; où le réglage d'éclairement d'au moins une partie du réseau d'éclairage directionnel (31a) dans le système d'éclairage (3) selon les informations de configuration du mode d'éclairage personnalisé, comprend : le fait de passer du mode d'éclairage par défaut au mode d'éclairage personnalisé ; régler l'éclairement du réseau d'éclairage directionnel (31a) selon les informations de configuration du mode d'éclairage personnalisé ; et régler l'éclairement et la température de couleur du réseau d'éclairage directionnel (31a) selon les informations de configuration du mode d'éclairage par défaut, jusqu'à ce qu'une période d'exécution prédéfinie dans l'instruction de l'utilisateur soit dépassée ou qu'une instruction d'annulation soit reçue ; ou
le réseau d'éclairage directionnel (31a) qui est réglé selon les informations de configuration du mode d'éclairage par défaut, est un réseau d'éclairage directionnel de longue durée ; et le réseau d'éclairage directionnel (31a) qui est réglé selon les informations de configuration du mode d'éclairage personnalisé, est un réseau d'éclairage directionnel de courte durée ; où le réseau d'éclairage directionnel de longue durée et le réseau d'éclairage directionnel de courte durée sont différents ; où le réglage de l'éclairement d'au moins une partie du réseau d'éclairage directionnel dans le système d'éclairage (3) selon les informations de configuration du mode d'éclairage personnalisé, comprend : le fait de régler l'éclairement du réseau d'éclairage directionnel de courte durée dans le système d'éclairage (3) selon les informations de configuration du mode d'éclairage personnalisé, jusqu'à ce qu'une période d'exécution prédéfinie dans l'instruction de l'utilisateur soit dépassée ou qu'une instruction d'annulation soit reçue.
